# EUROPEAN PATENT SPECIFICATION

(11) **EP 1 124 855 B1**
(45) Date of publication and mention of the grant of the patent: **19.08.2009**
(21) Application number: 99965734.9
(22) Date of filing: 25.10.1999
(51) Int. Cl.: C07K 16/28, C07K 14/47, C12N 5/12, A61K 39/395, A61K 38/17, C12N 15/866, C12N 5/10, G01N 33/577, G01N 33/68, C12N 15/11, A61P 13/12

(54) **COMPOSITIONS AND METHODS FOR TREATING POLYCYSTIC KIDNEY DISEASE**
ZUSAMMENSETZUNGEN UND VERFAHREN ZUR BEHANDLUNG DER POLYZYSTISCHEN NIERENKRANKHEIT
COMPOSITIONS ET PROCEDES POUR LE TRAITEMENT D'UNE POLYKYSTOSE RENALE

(30) Priority: 26.10.1998 US 105731 P; 27.10.1998 US 105876 P; 25.06.1999 US 141175 P
(43) Date of publication of application: 22.08.2001
(73) Proprietor: GENZYME CORPORATION, Framingham, MA 01701-9322 (US)
(72) Inventor: IBRAGHIMOV-BESKROVNAYA, Oxana, Southborough - MA 01772 (US); VAN-DELLEN, K., Framingham, MA 01701 (US); PETRY, Linda, R., Framingham, MA 01701 (US)
(74) Representative: Gates, Marie Christina Esther
(86) International application number: PCT/US1999/025091
(87) International publication number: WO 2000/024780

(56) References cited:
- WO-A-95/34573
- VAN ADELSBERG J ET AL: "Polycystin expression is temporally and spatially regulated during renal development." AMERICAN JOURNAL OF PHYSIOLOGY, (1997) 272 F602-F609, XP000892142
- PALSSON R ET AL: "Characterization and cell distribution of polycystin, the product of autosomal dominant polycystc kidney disease gene 1." MOLECULAR MEDICINE, (1996) 2 702-11, XP000892141

### SEQUENCE LISTING

<110> Genzyme Corporation
<120> COMPOSITIONS AND METHODS FOR IDENTIFYING AND TREATING POLYCYSTIC KIDNEY DISEASE
<130> 126881206140
<140> PCT/US99/
   <141> 1999-10-22
<150> 60/105,731
   <151> 1998-10-28
<150> 60/105,876
   <151> 1998-10-27
<150> 60/141,175
   <151> 1999-06-25
<160> 3
<170> FastSEQ for Windows Version 3.0
<210> 1
   <211> 14060
   <212> DNA
   <213> Homo sapiens
<220>
   <221> CDS
   <222> (135)...(13040)
<221> misc_feature
   <222> (2621)...(2710)
   <223> epitope in the loop region of the polycystin transmembrane domain
<221> misc_feature
   <222> (2734)...(3094)
   <223> epitope in the loop region of the polycystin transmembrane domain
<221> misc_feature
   <222> (3166)...(3300)
   <223> epitope in the loop region of the polycystin transmembrane domain
<221> misc_feature
   <222> (3364)...(3578)
   <223> epitope in the loop region of the polycystin transmembrane domain
<221> misc_feature
   <222> (3623)...(3688)
   <223> epitope in the loop region of the polycystin transmembrane domain
<221> misc_feature
   <222> (3710)...(3914)
   <223> epitope in the loop region of the polycystin transmembrane domain
<221> misc_feature
   <222> (3931)...(4046)
   <223> epitope in the loop region of the polycystin transmembrane domain
<221> misc_feature
   <222> (2166)...(2599) <223> epitope outside the loop region but within the polycystin transmembrane domain
<221> misc_feature
   <222> (4097)...(4302)
   <223> epitope in the loop region of the polycystin transmembrane domain
<221> misc_feature
   <222> (4148)...(4219)
   <223> epitope in the loop region of the polycystin transmembrane domain
<221> misc_feature
   <222> (4220)...(4302)
   <223> epitope in the loop region of the polycystin transmembrane domain
<221> misc_feature
   <222> (27)...(360)
   <223> epitope in the loop region of the polycystin transmembrane domain
<221> misc_feature
   <222> (843)...(1200)
   <223> Ig-like domain of polycystin
<221> misc_feature
   <222> (1205)...(1625)
   <223> Ig-like domain of polycystin
<221> misc_feature
   <222> (1626)...(2136)
   <223> Ig-like domain of polycystin
<221> misc_feature
   <222> (2166)...(2599)
   <223> isolated polypeptide
<400> 1
   gctcagcagc aggtcgcggc cgcagcccca tccagcccgc gcccgccatg ccgtccgcgg     60
   gccccgcctg agctgcggtc tccgcgcgcg ggcgggcctg gggacggcgg ggccatgcgc    120
   gcgctgccct aacg atg ccg ccc gcc gcg ccc gcc cgc ctg gcg ctg gcc      170
   ctg ggc ctg ggc ctg tgg ctc ggg gcg ctg gcg ggg ggc ccc ggg cgc      218
   ggc tgc ggg ccc tgc gag ccc ccc tgc ctc tgc ggc cca gcg ccc ggc      266
   gcc gcc tgc cgc gtc aac tgc tcg ggc cgc ggg ctg cgg acg ctc ggt      314
   ccc gcg ctg cgc atc ccc gcg gac gcc aca gcg cta gac gtc tcc cac      362
   aac ctg ctc cgg gcg ctg gac gtt ggg ctc ctg gcg aac ctc tcg gcg 410
   ctg gca gag ctg gat ata agc aac aac aag att tct acg tta gaa gaa 458
   gga ata ttt gct aat tta ttt aat tta agt gaa ata aac ctg agt ggg 506
   aac ccg ttt gag tgt gac tgt ggc ctg gcg tgg ctg ccg cga tgg gcg 554
   gag gag cag cag gtg cgg gtg gtg cag ccc gag gca gcc acg tgt gct 602
   ggg cct ggc tcc ctg gct ggc cag cct ctg ctt ggc atc ccc ttg ctg 650
   gac agt ggc tgt ggt gag gag tat gtc gcc tgc ctc cct gac aac agc 698
   tca ggc acc gtg gca gca gtg tcc ttt tca gct gcc cac gaa ggc ctg 746
   ctt cag cca gag gcc tgc agc gcc ttc tgc ttc tcc acc ggc cag ggc 794
   ctc gca gcc ctc tcg gag cag ggc tgg tgc ctg tgt ggg gcg gcc cag 842
   ccc tcc agt gcc tcc ttt gcc tgc ctg tcc ctc tgc tcc ggc ccc ccg 890
   cca cct cct gcc ccc acc tgt agg ggc ccc acc ctc ctc cag cac gtc 938
   ttc cct gcc tcc cca ggg gcc acc ctg gtg ggg ccc cac gga cct ctg 986
   gcc tct ggc cag cta gca gcc ttc cac atc gct gcc ccg ctc cct gtc 1034
   act gcc aca cgc tgg gac ttc gga gac ggc tcc gcc gag gtg gat gcc 1082
   gct ggg ccg gct gcc tcg cat cgc tat gtg ctg cct ggg cgc tat cac 1130
   gtg acg gcc gtg ctg gcc ctg ggg gcc ggc tca gcc ctg ctg ggg aca      1178
   gac gtg cag gtg gaa gcg gca cct gcc gcc ctg gag ctc gtg tgc ccg      1226
   tcc tcg gtg cag agt gac gag agc ctt gac ctc agc atc cag aac cgc      1274
   ggt ggt tca ggc ctg gag gcc gcc tac agc atc gtg gcc ctg ggc gag      1322
   gag ccg gcc cga gcg gtg cac ccg ctc tgc ccc tcg gac acg gag atc      1370
   ttc cct ggc aac ggg cac tgc tac cgc ctg gtg gtg gag aag gcg gcc      1418
   tgg ctg cag gcg cag gag cag tgt cag gcc tgg gcc ggg gcc gcc ctg      1466
   gca atg gtg gac agt ccc gcc gtg cag cgc ttc ctg gtc tcc cgg gtc      1514
   acc agg agc cta gac gtg tgg atc ggc ttc tcg act gtg cag ggg gtg      1562
   gag gtg ggc cca gcg ccg cag ggc gag gcc ttc agc ctg gag agc tgc      1610
   cag aac tgg ctg ccc ggg gag cca cac cca gcc aca gcc gag cac tgc      1658
   gtc cgg ctc ggg ccc acc ggg tgg tgt aac acc gac ctg tgc tca gcg      1706
   ccg cac agc tac gtc tgc gag ctg cag ccc gga ggc cca gtg cag gat      1754
   gcc gag aac ctc ctc gtg gga gcg ccc agt ggg gac ctg cag gga ccc      1802
   ctg acg cct ctg gca cag cag gac ggc ctc tca gcc ccg cac gag ccc      1850
   gtg gag gtc atg gta ttc ccg ggc ctg cgt ctg agc cgt gaa gcc ttc      1898
   ctc acc acg gcc gaa ttt ggg acc cag gag ctc cgg cgg ccc gcc cag      1946
   ctg cgg ctg cag gtg tac cgg ctc ctc agc aca gca ggg acc ccg gag      1994
   aac ggc agc gag cct gag agc agg tcc ccg gac aac agg acc cag ctg      2042
   gcc ccc gcg tgc atg cca ggg gga cgc tgg tgc cct gga gcc aac atc      2090
   tgc ttg ccg ctg gac gcc tct tgc cac ccc cag gcc tgc gcc aat ggc      2138
   tgc acg tca ggg cca ggg cta ccc ggg gcc ccc tat gcg cta tgg aga      2186
   gag ttc ctc ttc tcc gtt gcc gcg ggg ccc ccc gcg cag tac tcg gtc      2234
   acc ctc cac ggc cag gat gtc ctc atg ctc cct ggt gac ctc gtt ggc      2282
   ttg cag cac gac gct ggc cct ggc gcc ctc ctg cac tgc tcg ccg gct      2330
   ccc ggc cac cct ggt ccc cag gcc ccg tac ctc tcc gcc aac gcc tcg      2378
   tca tgg ctg ccc cac ttg cca gcc cag ctg gag ggc act tgg gcc tgc      2426
   cct gcc tgt gcc ctg cgg ctg ctt gca gcc acg gaa cag ctc acc gtg      2474
   ctg ctg ggc ttg agg ccc aac cct gga ctg cgg atg cct ggg cgc tat      2522
   gag gtc cgg gca gag gtg ggc aat ggc gtg tcc agg cac aac ctc tcc      2570
   tgc agc ttt gac gtg gtc tcc cca gtg gct ggg ctg cgg gtc atc tac      2618
   cct gcc ccc cgc gac ggc cgc ctc tac gtg ccc acc aac ggc tca gcc      2666
   ttg gtg ctc cag gtg gac tct ggt gcc aac gcc acg gcc acg gct cgc      2714
   tgg cct ggg ggc agt gtc agc gcc cgc ttt gag aat gtc tgc cct gcc      2762
   ctg gtg gcc acc ttc gtg ccc ggc tgc ccc tgg gag acc aac gat acc      2810
   ctg ttc tca gtg gta gca ctg ccg tgg ctc agt gag ggg gag cac gtg      2858
   gtg gac gtg gtg gtg gaa aac agc gcc agc cgg gcc aac ctc agc ctg      2906
   cgg gtg acg gcg gag gag ccc atc tgt ggc ctc cgc gcc acg ccc agc      2954
   ccc gag gcc cgt gta ctg cag gga gtc cta gtg agg tac agc ccc gtg      3002
   gtg gag gcc ggc tcg gac atg gtc ttc cgg tgg acc atc aac gac aag      3050
   cag tcc ctg acc ttc cag aac gtg gtc ttc aat gtc att tat cag agc      3098
   gcg gcg gtc ttc aag ctc tca ctg acg gcc tcc aac cac gtg agc aac      3146
   gtc acc gtg aac tac aac gta acc gtg gag cgg atg aac agg atg cag      3194
   ggt ctg cag gtc tcc aca gtg ccg gcc gtg ctg tcc ccc aat gcc acg      3242
   cta gca ctg acg gcg ggc gtg ctg gtg gac tcg gcc gtg gag gtg gcc      3290
   ttc ctg tgg aac ttt ggg gat ggg gag cag gcc ctc cac cag ttc cag      3338
   cct ccg tac aac gag tcc ttc ccg gtt cca gac ccc tcg gtg gcc cag      3386
   gtg ctg gtg gag cac aat gtc atg cac acc tac gct gcc cca ggt gag      3434
   tac ctc ctg acc gtg ctg gca tct aat gcc ttc gag aac ctg acg cag      3482
   cag gtg cct gtg agc gtg cgc gcc tcc ctg ccc tcc gtg gct gtg ggt      3530
   gtg agt gac ggc gtc ctg gtg gcc ggc cgg ccc gtc acc ttc tac ccg 3578
   cac ccg ctg ccc tcg cct ggg ggt gtt ctt tac acg tgg gac ttc ggg 3626
   gac ggc tcc cct gtc ctg acc cag agc cag ccg gct gcc aac cac acc 3674
   tat gcc tcg agg ggc acc tac cac gtg cgc ctg gag gtc aac aac acg 3722
   gtg agc ggt gcg gcg gcc cag gcg gat gtg cgc gtc ttt gag gag ctc 3770
   cgc gga ctc agc gtg gac atg agc ctg gcc gtg gag cag ggc gcc ccc 3818
   gtg gtg gtc agc gcc gcg gtg cag acg ggc gac aac atc acg tgg acc 3866
   ttc gac atg ggg gac ggc acc gtg ctg tcg ggc ccg gag gca aca gtg 3914
   gag cat gtg tac ctg cgg gca cag aac tgc aca gtg acc gtg ggt gcg 3962
   gcc agc ccc gcc ggc cac ctg gcc cgg agc ctg cac gtg ctg gtc ttc 4010
   gtc ctg gag gtg ctg cgc gtt gaa ccc gcc gcc tgc atc ccc acg cag 4058
   cct gac gcg cgg ctc acg gcc tac gtc acc ggg aac ccg gcc cac tac 4106
   ctc ttc gac tgg acc ttc ggg gat ggc tcc tcc aac acg acc gtg cgg 4154
   ggg tgc ccg acg gtg aca cac aac ttc acg cgg agc ggc acg ttc ccc 4202
   ctg gcg ctg gtg ctg tcc agc cgc gtg aac agg gcg cat tac ttc acc 4250
   agc atc tgc gtg gag cca gag gtg ggc aac gtc acc ctg cag cca gag 4298
   agg cag ttt gtg cag ctc ggg gac gag gcc tgg ctg gtg gca tgt gcc      4346
   tgg ccc ccg ttc ccc tac cgc tac acc tgg gac ttt ggc acc gag gaa      4394
   gcc gcc ccc acc cgt gcc agg ggc cct gag gtg acg ttc atc tac cga      4442
   gac cca ggc tcc tat ctt gtg aca gtc acc gcg tcc aac aac atc tct      4490
   gct gcc aat gac tca gcc ctg gtg gag gtg cag gag ccc gtg ctg gtc      4538
   acc agc atc aag gtc aat ggc tcc ctt ggg ctg gag ctg cag cag ccg      4586
   tac ctg ttc tct gct gtg ggc cgt ggg cgc ccc gcc agc tac ctg tgg      4634
   gat ctg ggg gac ggt ggg tgg ctc gag ggt ccg gag gtc acc cac gct      4682
   tac aac agc aca ggt gac ttc acc gtt agg gtg gcc ggc tgg aat gag      4730
   gtg agc cgc agc gag gcc tgg ctc aat gtg acg gtg aag cgg cgc gtg      4778
   cgg ggg ctc gtc gtc aat gca agc cgc acg gtg gtg ccc ctg aat ggg      4826
   agc gtg agc ttc agc acg tcg ctg gag gcc ggc agt gat gtg cgc tat      4874
   tcc tgg gtg ctc tgt gac cgc tgc acg ccc atc cct ggg ggt cct acc      4922
   atc tct tac acc ttc cgc tcc gtg ggc acc ttc aat atc atc gtc acg      4970
   gct gag aac gag gtg ggc tcc gcc cag gac agc atc ttc gtc tat gtc      5018
   ctg cag ctc ata gag ggg ctg cag gtg gtg ggc ggt ggc cgc tac ttc      5066
   ccc acc aac cac acg gta cag ctg cag gcc gtg gtt agg gat ggc acc      5114
   aac gtc tcc tac agc tgg act gcc tgg agg gac agg ggc ccg gcc ctg      5162
   gcc ggc agc ggc aaa ggc ttc tcg ctc acc gtg ctc gag gcc ggc acc      5210
   tac cat gtg cag ctg cgg gcc acc aac atg ctg ggc agc gcc tgg gcc      5258
   gac tgc acc atg gac ttc gtg gag cct gtg ggg tgg ctg atg gtg acc      5306
   gcc tcc ccg aac cca gct gcc gtc aac aca agc gtc acc ctc agt gcc      5354
   gag ctg gct ggt ggc agt ggt gtc gta tac act tgg tcc ttg gag gag      5402
   ggg ctg agc tgg gag acc tcc gag cca ttt acc acc cat agc ttc ccc      5450
   aca ccc ggc ctg cac ttg gtc acc atg acg gca ggg aac ccg ctg ggc      5498
   tca gcc aac gcc acc gtg gaa gtg gat gtg cag gtg cct gtg agt ggc      5546
   ctc agc atc agg gcc agc gag ccc gga ggc agc ttc gtg gcg gcc ggg      5594
   tcc tct gtg ccc ttt tgg ggg cag ctg gcc acg ggc acc aat gtg agc      5642
   tgg tgc tgg gct gtg ccc ggc ggc agc agc aag cgt ggc cct cat gtc      5690
   acc atg gtc ttc ccg gat gct ggc acc ttc tcc atc cgg ctc aat gcc      5738
   tcc aac gca gtc agc tgg gtc tca gcc acg tac aac ctc acg gcg gag      5786
   gag ccc atc gtg ggc ctg gtg ctg tgg gcc agc agc aag gtg gtg gcg      5834
   ccc ggg cag ctg gtc cat ttt cag atc ctg ctg gct gcc ggc tca gct      5882
   gtc acc ttc cgc ctg cag gtc ggc ggg gcc aac ccc gag gtg ctc ccc      5930
   ggg ccc cgt ttc tcc cac agc ttc ccc cgc gtc gga gac cac gtg gtg      5978
   agc gtg cgg ggc aaa aac cac gtg agc tgg gcc cag gcg cag gtg cgc      6026
   atc gtg gtg ctg gag gcc gtg agt ggg ctg cag atg ccc aac tgc tgc      6074
   gag cct ggc atc gcc acg ggc act gag agg aac ttc aca gcc cgc gtg      6122
   cag cgc ggc tct cgg gtc gcc tac gcc tgg tac ttc tcg ctg cag aag      6170
   gtc cag ggc gac tcg ctg gtc atc ctg tcg ggc cgc gac gtc acc tac      6218
   acg ccc gtg gcc gcg ggg ctg ttg gag atc cag gtg cgc gcc ttc aac      6266
   gcc ctg ggc agt gag aac cgc acg ctg gtg ctg gag gtt cag gac gcc      6314
   gtc cag tat gtg gcc ctg cag agc ggc ccc tgc ttc acc aac cgc tcg      6362
   gcg cag ttt gag gcc gcc acc agc ccc agc ccc cgg cgt gtg gcc tac      6410
   cac tgg gac ttt ggg gat ggg tcg cca ggg cag gac aca gat gag ccc      6458
   agg gcc gag cac tcc tac ctg agg cct ggg gac tac cgc gtg cag gtg      6506
   aac gcc tcc aac ctg gtg agc ttc ttc gtg gcg cag gcc acg gtg acc      6554
   gtc cag gtg ctg gcc tgc cgg gag ccg gag gtg gac gtg gtc ctg ccc      6602
   ctg cag gtg ctg atg cgg cga tca cag cgc aac tac ttg gag gcc cac      6650
   gtt gac ctg cgc gac tgc gtc acc tac cag act gag tac cgc tgg gag      6698
   gtg tat cgc acc gcc agc tgc cag cgg ccg ggg cgc cca gcg cgt gtg 6746
   gcc ctg ccc ggc gtg gac gtg agc cgg cct cgg ctg gtg ctg ccg cgg 6794
   ctg gcg ctg cct gtg ggg cac tac tgc ttt gtg ttt gtc gtg tca ttt 6842
   ggg gac acg cca ctg aca cag agc atc cag gcc aat gtg acg gtg gcc 6890
   ccc gag cgc ctg gtg ccc atc att gag ggt ggc tca tac cgc gtg tgg 6938
   tca gac aca cgg gac ctg gtg ctg gat ggg agc gag tcc tac gac ccc 6986
   aac ctg gag gac ggc gac cag acg ccg ctc agt ttc cac tgg gcc tgt 7034
   gtg gct tcg aca cag agg gag gct ggc ggg tgt gcg ctg aac ttt ggg 7082
   ccc cgc ggg agc agc acg gtc acc att cca cgg gag cgg ctg gcg gct 7130
   ggc gtg gag tac acc ttc agc ctg acc gtg tgg aag gcc ggc cgc aag 7178
   gag gag gcc acc aac cag acg gtg ctg atc cgg agt ggc cgg gtg ccc 7226
   att gtg tcc ttg gag tgt gtg tcc tgc aag gca cag gcc gtg tac gaa 7274
   gtg agc cgc agc tcc tac gtg tac ttg gag ggc cgc tgc ctc aat tgc 7322
   agc agc ggc tcc aag cga ggg cgg tgg gct gca cgt acg ttc agc aac 7370
   aag acg ctg gtg ctg gat gag acc acc aca tcc acg ggc agt gca ggc 7418
   atg cga ctg gtg ctg cgg cgg ggc gtg ctg cgg gac ggc gag gga tac 7466
   acc ttc acg ctc acg gtg ctg ggc cgc tct ggc gag gag gag ggc tgc      7514
   gcc tcc atc cgc ctg tcc ccc aac cgc ccg ccg ctg ggg ggc tct tgc      7562
   cgc ctc ttc cca ctg ggc gct gtg cac gcc ctc acc acc aag gtg cac      7610
   ttc gaa tgc acg ggc tgg cat gac gcg gag gat gct ggc gcc ccg ctg      7658
   gtg tac gcc ctg ctg ctg cgg cgc tgt cgc cag ggc cac tgc gag gag      7706
   ttc tgt gtc tac aag ggc agc ctc tcc agc tac gga gcc gtg ctg ccc      7754
   ccg ggt ttc agg cca cac ttc gag gtg ggc ctg gcc gtg gtg gtg cag      7802
   gac cag ctg gga gcc gct gtg gtc gcc ctc aac agg tct ttg gcc atc      7850
   acc ctc cca gag ccc aac ggc agc gca acg ggg ctc aca gtc tgg ctg      7898
   cac ggg ctc acc gct agt gtg ctc cca ggg ctg ctg cgg cag gcc gat      7946
   ccc cag cac gtc atc gag tac tcg ttg gcc ctg gtc acc gtg ctg aac      7994
   gag tac gag cgg gcc ctg gac gtg gcg gca gag ccc aag cac gag cgg      8042
   cag cac cga gcc cag ata cgc aag aac atc acg gag act ctg gtg tcc      8090
   ctg agg gtc cac act gtg gat gac atc cag cag atc gct gct gcg ctg      8138
   gcc cag tgc atg ggg ccc agc agg gag ctc gta tgc cgc tcg tgc ctg      8186
   aag cag acg ctg cac aag ctg gag gcc atg atg ctc atc ctg cag gca      8234
   gag acc acc gcg ggc acc gtg acg ccc acc gcc atc gga gac agc atc      8282
   ctc aac atc aca gga gac ctc atc cac ctg gcc agc tcg gac gtg cgg      8330
   gca cca cag ccc tca gag ctg gga gcc gag tca cca tct cgg atg gtg      8378
   gcg tcc cag gcc tac aac ctg acc tct gcc ctc atg cgc atc ctc atg      8426
   cgc tcc cgc gtg ctc aac gag gag ccc ctg acg ctg gcg ggc gag gag      8474
   atc gtg gcc cag ggc aag cgc tcg gac ccg cgg agc ctg ctg tgc tat      8522
   ggc ggc gcc cca ggg cct ggc tgc cac ttc tcc atc ccc gag gct ttc      8570
   agc ggg gcc ctg gcc aac ctc agt gac gtg gtg cag ctc atc ttt ctg      8618
   gtg gac tcc aat ccc ttt ccc ttt ggc tat atc agc aac tac acc gtc      8666
   tcc acc aag gtg gcc tcg atg gca ttc cag aca cag gcc ggc gcc cag      8714
   atc ccc atc gag cgg ctg gcc tca gag cgc gcc atc acc gtg aag gtg      8762
   ccc aac aac tcg gac tgg gct gcc cgg ggc cac cgc agc tcc gcc aac      8810
   tcc gcc aac tcc gtt gtg gtc cag ccc cag gcc tcc gtc ggt gct gtg      8858
   gtc acc ctg gac agc agc aac cct gcg gcc ggg ctg cat ctg cag ctc      8906
   aac tat acg ctg ctg gac ggc cac tac ctg tct gag gaa cct gag ccc      8954
   tac ctg gca gtc tac cta cac tcg gag ccc cgg ccc aat gag cac aac      9002
   tgc tcg gct agc agg agg atc cgc cca gag tca ctc cag ggt gct gac      9050
   cac cgg ccc tac acc ttc ttc att tcc ccg ggg agc aga gac cca gcg      9098
   ggg agt tac cat ctg aac ctc tcc agc cac ttc cgc tgg tcg gcg ctg      9146
   cag gtg tcc gtg ggc ctg tac acg tcc ctg tgc cag tac ttc agc gag      9194
   gag gac atg gtg tgg cgg aca gag ggg ctg ctg ccc ctg gag gag acc      9242
   tcg ccc cgc cag gcc gtc tgc ctc acc cgc cac ctc acc gcc ttc ggc      9290
   gcc agc ctc ttc gtg ccc cca agc cat gtc cgc ttt gtg ttt cct gag      9338
   ccg aca gcg gat gta aac tac atc gtc atg ctg aca tgt gct gtg tgc      9386
   ctg gtg acc tac atg gtc atg gcc gcc atc ctg cac aag ctg gac cag      9434
   ttg gat gcc agc cgg ggc cgc gcc atc cct ttc tgt ggg cag cgg ggc      9482
   cgc ttc aag tac gag atc ctc gtc aag aca ggc tgg ggc cgg ggc tca      9530
   ggt acc acg gcc cac gtg ggc atc atg ctg tat ggg gtg gac agc cgg      9578
   agc ggc cac cgg cac ctg gac ggc gac aga gcc ttc cac cgc aac agc      9626
   ctg gac atc ttc cgg atc gcc acc ccg cac agc ctg ggt agc gtg tgg      9674
   aag atc cga gtg tgg cac gac aac aaa ggg ctc agc cct gcc tgg ttc      9722
   ctg cag cac gtc atc gtc agg gac ctg cag acg gca cgc agc gcc ttc      9770
   ttc ctg gtc aat gac tgg ctt tcg gtg gag acg gag gcc aac ggg ggc      9818
   ctg gtg gag aag gag gtg ctg gcc gcg agc gac gca gcc ctt ttg cgc      9866
   ttc cgg cgc ctg ctg gtg gct gag ctg cag cgt ggc ttc ttt gac aag      9914
   cac atc tgg ctc tcc ata tgg gac cgg ccg cct cgt agc cgt ttc act      9962
   cgc atc cag agg gcc acc tgc tgc gtt ctc ctc atc tgc ctc ttc ctg     10010
   ggc gcc aac gcc gtg tgg tac ggg gct gtt ggc gac tct gcc tac agc     10058
   acg ggg cat gtg tcc agg ctg agc ccg ctg agc gtc gac aca gtc gct     10106
   gtt ggc ctg gtg tcc agc gtg gtt gtc tat ccc gtc tac ctg gcc atc     10154
   ctt ttt ctc ttc cgg atg tcc cgg agc aag gtg gct ggg agc ccg agc     10202
   ccc aca cct gcc ggg cag cag gtg ctg gac atc gac agc tgc ctg gac     10250
   tcg tcc gtg ctg gac agc tcc ttc ctc acg ttc tca ggc ctc cac gct     10298
   gag gcc ttt gtt gga cag atg aag agt gac ttg ttt ctg gat gat tct     10346
   aag agt ctg gtg tgc tgg ccc tcc ggc gag gga acg ctc agt tgg ccg     10394
   gac ctg ctc agt gac ccg tcc att gtg ggt agc aat ctg cgg cag ctg     10442
   gca cgg ggc cag gcg ggc cat ggg ctg ggc cca gag gag gac ggc ttc     10490
   tcc ctg gcc agc ccc tac tcg cct gcc aaa tcc ttc tca gca tca gat     10538
   gaa gac ctg atc cag cag gtc ctt gcc gag ggg gtc agc agc cca gcc     10586
   cct acc caa gac acc cac atg gaa acg gac ctg ctc agc agc ctg tcc     10634
   agc act cct ggg gag aag aca gag acg ctg gcg ctg cag agg ctg ggg 10682
   gag ctg ggg cca ccc agc cca ggc ctg aac tgg gaa cag ccc cag gca 10730
   gcg agg ctg tcc agg aca gga ctg gtg gag ggt ctg cgg aag cgc ctg 10778
   ctg ccg gcc tgg tgt gcc tcc ctg gcc cac ggg ctc agc ctg ctc ctg 10826
   gtg gct gtg gct gtg gct gtc tca ggg tgg gtg ggt gcg agc ttc ccc 10874
   ccg ggc gtg agt gtt gcg tgg ctc ctg tcc agc agc gcc agc ttc ctg 10922
   gcc tca ttc ctc ggc tgg gag cca ctg aag gtc ttg ctg gaa gcc ctg 10970
   tac ttc tca ctg gtg gcc aag cgg ctg cac ccg gat gaa gat gac acc 11018
   ctg gta gag agc ccg gct gtg acg cct gtg agc gca cgt gtg ccc cgc 11066
   gta cgg cca ccc cac ggc ttt gca ctc ttc ctg gcc aag gaa gaa gcc 11114
   cgc aag gtc aag agg cta cat ggc atg ctg cgg agc ctc ctg gtg tac 11162
   atg ctt ttt ctg ctg gtg acc ctg ctg gcc agc tat ggg gat gcc tca 11210
   tgc cat ggg cac gcc tac cgt ctg caa agc gcc atc aag cag gag ctg 11258
   cac agc cgg gcc ttc ctg gcc atc acg cgg tct gag gag ctc tgg cca 11306
   tgg atg gcc cac gtg ctg ctg ccc tac gtc cac ggg aac cag tcc agc 11354
   cca gag ctg ggg ccc cca cgg ctg cgg cag gtg cgg ctg cag gaa gca 11402
   ctc tac cca gac cct ccc ggc ccc agg gtc cac acg tgc tcg gcc gca 11450
   gga ggc ttc agc acc agc gat tac gac gtt ggc tgg gag agt cct cac    11498
   aat ggc tcg ggg acg tgg gcc tat tca gcg ccg gat ctg ctg ggg gca    11546
   tgg tcc tgg ggc tcc tgt gcc gtg tat gac agc ggg ggc tac gtg cag    11594
   gag ctg ggc ctg agc ctg gag gag agc cgc gac cgg ctg cgc ttc ctg    11642
   cag ctg cac aac tgg ctg gac aac agg agc cgc gct gtg ttc ctg gag    11690
   ctc acg cgc tac agc ccg gcc gtg ggg ctg cac gcc gcc gtc acg ctg    11738
   cgc ctc gag ttc ccg gcg gcc ggc cgc gcc ctg gcc gcc ctc agc gtc    11786
   cgc ccc ttt gcg ctg cgc cgc ctc agc gcg ggc ctc tcg ctg cct ctg    11834
   ctc acc tcg gtg tgc ctg ctg ctg ttc gcc gtg cac ttc gcc gtg gcc    11882
   gag gcc cgt act tgg cac agg gaa ggg cgc tgg cgc gtg ctg cgg ctc    11930
   gga gcc tgg gcg cgg tgg ctg ctg gtg gcg ctg acg gcg gcc acg gca    11978
   ctg gta cgc ctc gcc cag ctg ggt gcc gct gac cgc cag tgg acc cgt    12026
   ttc gtg cgc ggc cgc ccg cgc cgc ttc act agc ttc gac cag gtg gcg    12074
   cag ctg agc tcc gca gcc cgt ggc ctg gcg gcc tcg ctg ctc ttc ctg    12122
   ctt ttg gtc aag gct gcc cag cag cta cgc ttc gtg cgc cag tgg tcc    12170
   gtc ttt ggc aag aca tta tgc cga gct ctg cca gag ctc ctg ggg gtc    12218
   acc ttg ggc ctg gtg gtg ctc ggg gta gcc tac gcc cag ctg gcc atc     12266
   ctg ctc gtg tct tcc tgt gtg gac tcc ctc tgg agc gtg gcc cag gcc     12314
   ctg ttg gtg ctg tgc cct ggg act ggg ctc tct acc ctg tgt cct gcc     12362
   gag tcc tgg cac ctg tca ccc ctg ctg tgt gtg ggg ctc tgg gca ctg     12410
   cgg ctg tgg ggc gcc cta cgg ctg ggg gct gtt att ctc cgc tgg cgc     12458
   tac cac gcc ttg cgt gga gag ctg tac cgg ccg gcc tgg gag ccc cag     12506
   gac tac gag atg gtg gag ttg ttc ctg cgc agg ctg cgc ctc tgg atg     12554
   ggc ctc agc aag gtc aag gag ttc cgc cac aaa gtc cgc ttt gaa ggg     12602
   atg gag ccg ctg ccc tct cgc tcc tcc agg ggc tcc aag gta tcc ccg     12650
   gat gtg ccc cca ccc agc gct ggc tcc gat gcc tcg cac ccc tcc acc     12698
   tcc tcc agc cag ctg gat ggg ctg agc gtg agc ctg ggc cgg ctg ggg     12746
   aca agg tgt gag cct gag ccc tcc cgc ctc caa gcc gtg ttc gag gcc     12794
   ctg ctc acc cag ttt gac cga ctc aac cag gcc aca gag gac gtc tac     12842
   cag ctg gag cag cag ctg cac agc ctg caa ggc cgc agg agc agc cgg     12890
   gcg ccc gcc gga tct tcc cgt ggc cca tcc ccg ggc ctg cgg cca gca     12938
   ctg ccc agc cgc ctt gcc cgg gcc agt cgg ggt gtg gac ctg gcc act     12986
   ggc ccc agc agg aca ccc ctt cgg gcc aag aac aag gtc cac ccc agc     13034
   agc act tagtcctcct tcctggcggg ggtgggccgt ggagtcggag tggacaccgc   13090
   tcagtattac tttctgccgc tgtcaaggcc gagggccagg cagaatggct gcacgtaggt  13150
   tccccagaga gcaggcaggg gcatctgtct gtctgtgggc ttcagcactt taaagaggct  13210
   gtgtggccaa ccaggaccca gggtcccctc cccagctccc ttgggaagga cacagcagta  13270
   ttggacggtt tctagcctct gagatgctaa tttatttccc cgagtcctca ggtacagcgg  13330
   gctgtgcccg gccccacccc ctgggcagat gtcccccact gctaaggctg ctggcttcag  13390
   ggagggttag cctgcaccgc cgccaccctg cccctaagtt attacctctc cagttcctac  13450
   cgtactccct gcaccgtctc actgtgtgtc tcgtgtcagt aatttatatg gtgttaaaat  13510
   gtgtatattt ttgtatgtca ctattttcac tagggctgag gggcctgcgc ccagagctgg  13570
   cctcccccaa cacctgctgc gcttggtagg tgtggtggcg ttatggcagc ccggctgctg  13630
   cttggatgcg agcttggcct tgggccggtg ctgggggcac agctgtctgc caggcactct  13690
   catcacccca gaggccttgt catcctccct tgccccaggc caggtagcaa gagagcagcg  13750
   cccaggcctg ctggcatcag gtctgggcaa gtagcaggac taggcatgtc agaggacccc  13810
   agggtggtta gaggaaaaga ctcctcctgg gggctggctc ccagggtgga ggaaggtgac  13870
   tgtgtgtgtg tgtgtgtgcg cgcgcgcacg cgcgagtgtg ctgtatggcc caggcagcct  13930
   caaggccctc ggagctggct gtgcctgctt ctgtgtacca cttctgtggg catggccgct  13990
   tctagagcct cgacaccccc ccaacccccg caccaagcag acaaagtcaa taaaagagct  14050
   gtctgactgc                                                         14060
<210> 2
   <211> 4302
   <212> PRT
   <213> Homo sapiens
<400> 2
<210> 3
   <211> 34
   <212> DNA
   <213> Homo sapiens
<400> 3
   gtaatacgac tcactatagg gcgagccacc atgg 34

## Description

### TECHNICAL FIELD

This invention is in the field of nephrology. The compositions and methods of the present invention are particularly useful in diagnoses and treatment of polycystic renal diseases.

### BACKGROUND OF THE INVENTION

Polycystic kidney disease (PKD) is a common inherited condition for which there are no cures and few effective therapies. The disease can be transmitted as an autosomal dominant or recessive defect. The dominant form of PKD is one of the most prevalent life-threatening genetic diseases, affecting approximately 600,000 Americans and more than 12 million families worldwide. The National Institutes of Health estimates that one in 400 to 1,000 persons has autosomal dominant polycystic kidney disease (ADPKD), and one in 10,000 to 40,000 individuals has autosomal recessive polycystic kidney disease (ARPKD). More than fifty percent of the affected individuals are expected to develop renal failure by the age of 60; consequently, ADPKD currently accounts for 4 to 8 percent of the renal dialysis and transplantation cases in the United States and Europe (Robinson and Hawkins (1981) Proc. European Dialysis and Transplant Assn. 17:20).

Most forms of PKD are characterized by the development of fluid-filled cysts from the nephrons and collecting ducts of affected kidney tissue, which results in grossly enlarged kidneys with progressively weakened renal-concentration ability. Cyst development can also occur in other ductal organs such as liver, pancreas and spleen. Further systemic manifestations may include gastrointestinal, cardiovascular, and musculoskeletal abnormalities, such as colonic diverticulitis, berry aneurysms, hernias, and mitral valve prolapse (Gabow, et al. (1989) Adv. Nephrol. 18:19-32 and Gabow et al. (1993) New Eng. J. Med. 329:332-342). Hypertension and endocrine dysfunction are also common in ADPKD patients, appearing even before symptoms of renal insufficiency.

Recently, a few genetic attributes of PKD have been identified. Linkage studies and mutation analysis have indicated a causative gene (PKD1) located on chromosome 16p13.3, which is responsible for eighty-five percent of ADPKD cases (Reeders et al. (1985) Nature 317:542-544; Breuning et al. (1987) Lancet ii; 1359- 1361). A large number of mutations in the PKD1 gene sequences have been found to be associated with the onset of polycystic kidney disease. Apart from large genomic deletions that eliminate PKD1, the mutations that have been defined clearly in ADPKD1 families appear to result in the transcription of a truncated or abnormal message RNA from the affected allele (The American PKD1 Consortium (1995) Human Mol. Genet. 4:575-582). These gene sequence alterations include small in-frame deletions, deletions and missense mutations that result in premature termination, splice-site mutations and chromosomal translocations which interrupt the gene. Most of the other ADPKD cases can be attributed to PKD2 (Kimberling W.J. et al. (1993) Genomics 18:467-472; Mochizuki T. et al. (1996) Science 272:1339-1342), with less than one percent due to the third locus for ADPKD, which has not been mapped yet.

The wild-type PKD1 gene encodes a large protein, polycystin-1, which is predicted to be approximately 462 kD in size. The primary sequence of polycystin predicts a protein having structural features characteristic of a cell surface receptor or adhesion molecule. At the N-terminus, an extracellular domain of about 3,000 amino acids contains a number of recognizable protein motifs known for their involvement in protein-protein interaction. At the C-terminus, a short cytosolic domain consisting of approximately 250 amino acids possess several phosphorylation sites and a potential PEST (proline, glutamic acid, serine, and threonine) sequence. Linking the two terminal regions is the transmembrane domain of about 1,000 amino acids in length that comprises a group of characteristic seven membrane segments also found in the G-protein coupled cell surface receptors.

Highly conserved motifs residing in the N-terminal extracellular domain include two leucine-rich repeats (LRRs) with cysteine-rich flanking regions, immunoglobulin (Ig)-like repeats, and a C-type lectin domain. Leucine-rich repeats (LRRs) are commonly found in the leucine-rich glycoprotein family, which takes part in a diversity of physiological events. Proteins sharing this homology include but are not limited to α2-glycoprotein, members of the GPIb.LX complex (von Willebrand factor receptor), *Drosophila chaoptin,* toll and slit (Bums et al. (1995) Human Mol. Genet. 4:575-82). Many LRR proteins are localized in the plasma membrane or extracellular matrix and are thought to be involved in cell adhesion and developmental regulation (Kobe et al. (1994) Trends Biochem. Sci. 19:415-21). At least half of the LRR-containing proteins identified thus far have been shown to be involved in signal transduction, as for example the receptor tyrosine kinases Trk, TrkB, and TrkC. In addition, C-type lectin domains are known to mediate calcium-dependent, carbohydrate binding in cell-cell and cell-matrix adhesion (The International Polycystic Kidney Disease Consortium (1995) Cell 81:289-98).

The 16 Ig-like domains are linearly segmented within the sequence such that the first Ig-like domain is localized between the LRRs and the C-type lectin domain while the remaining 15 Ig-like domains are tandemly clustered in the middle part of the molecule. Originally thought to be members of the Ig superfamily, recent work suggests that while PKD domains contain an Ig-like fold, they represent a novel family (Bycroft M. et al. (1999) EMBO J. 18:297-305).

Elucidation of the biological functions of a gene often begins with examining the expression pattern of the gene product. Polyclonal and monoclonal antibodies directed against peptide or fusion proteins, mainly from the C-terminal region of polycystin, have been used to study the expression of polycystin in human and animal tissues (Ward et al. (1996) Proc. Natl. Acad. Sci. USA 93:1524-1528; Griffin et al. (1996) Proc. Assoc. Am. Physicians 108:185-197; Peters et al. (1996) Lab. Invest. 75:221-230; Geng et al. (1996) J. Clin. Invest. 98:2674-2682; Paulson et al. (1996) Molec. Med. 2:702-711; Van Adelsberg et al. (1997) Am. J. Physiol. 272:F602-F609; Ibraghimov-Beskrovnaya et al. (1997) Proc. Natl. Acad. Sci. USA 94:6397-6402; Geng et al. (1997) Am. J. Physiol. 272:F451-F459; Griffin et al. (1997) Kidney Int. 52:1196-1205; Geng et al. (1997) J. Am. Soc. Nephrol. 8:372A). These studies indicate that polycystin is expressed in many tissues in addition to the kidney and the liver. These include the epithelial cells of pancreatic and mammary ducts, intestinal crypts, urothelium and bronchioles; basal keratinocytes of the skin; neural crest, brain, neural plexuses and adrenal medulla; myocardium vascular smooth muscle of elastic and distributive arteries; and certain endothelial cells (Griffin et al. (1996) Proc. Assoc. Am. Physicians 108:185-197; Geng et al. (1996) J. Clin. Invest. 98:2674-2682; Ibraghimov-Beskrovnaya et al. (1997) Proc. Natl. Acad. Sci. USA 94:6397-6402; Geng et al. (1997) Am. J. Physiol. 272:F451-F459; Griffin et al. (1997) Kidney Int. 52:1196-1205; Griffin et al. (1997) J. Am. Soc. Nephrol. 8:616-626; O'Sullivan et al. (1997) J. Am. Soc. Nephrol. 8:376A). Studies on the immunolocalization of polycystin in the kidney, however, yielded ambiguous results. For instance, there are conflicting observations as to whether polycystin is expressed in the glomeruli region of the kidney nephrons. There are also differing views as to whether polycystin is localized to basal and apical membranes of renal epithelial cells, and to what degree it is present in the cytoplasm.

There thus remains a considerable need for antibodies that specifically bind to endogenous polycystin and/or polycystin-related proteins for better characterization of their tissue distribution and intracellular localization. The generation of these antibodies would provide a significant contribution to elucidation of the basic biochemical mechanisms underlying the polycystic kidney disease; it would also greatly facilitate diagnosis, prognosis, and development of new and effective therapeutics for ADPKD. This invention satisfies these needs and provides related advantages as well.

### DISCLOSURE OF THE INVENTION

This invention provides an isolated antibody or a fragment thereof that binds to an epitope present in the transmembrane domain of polycystin and specifically recognizes at least one novel, polycystin-related polypeptide(s) (referred to herein as "PRP" for polycystin-related polypeptide) having an apparent molecular weight in the range of about 600 to about 800 kD. The invention also provides polynucleotides, polypeptides, gene delivery vehicles and host cells useful for generating such antibodies, as well as methods for using the antibodies and/or polypeptides for diagnostic purposes,

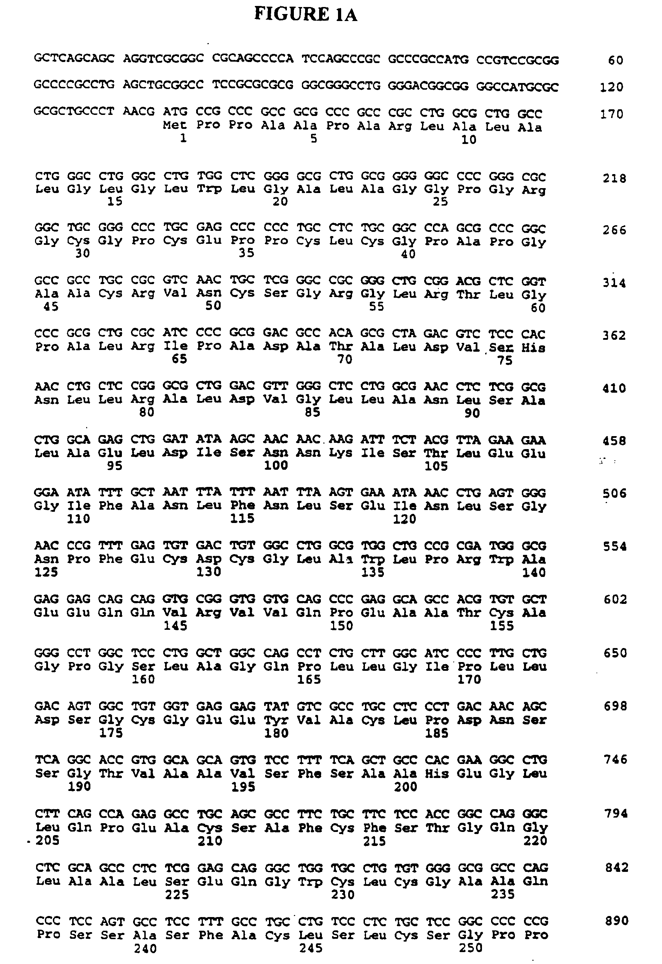

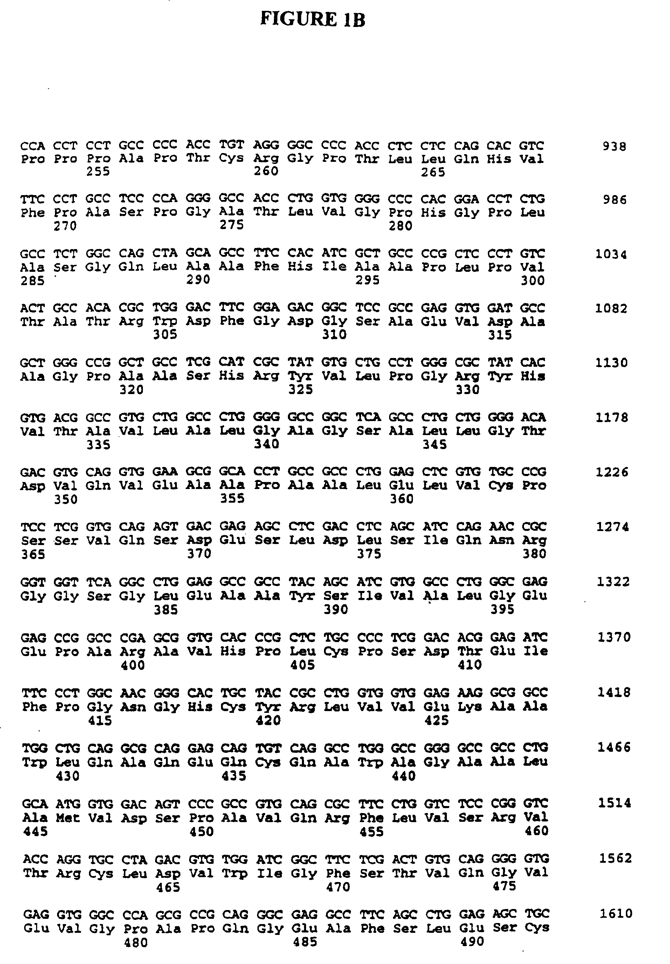

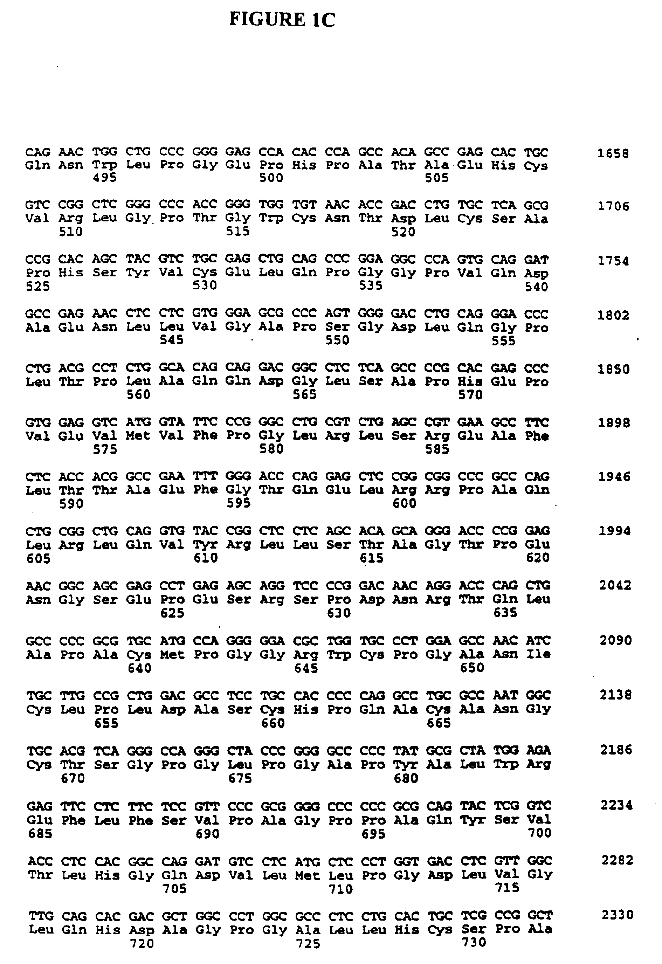

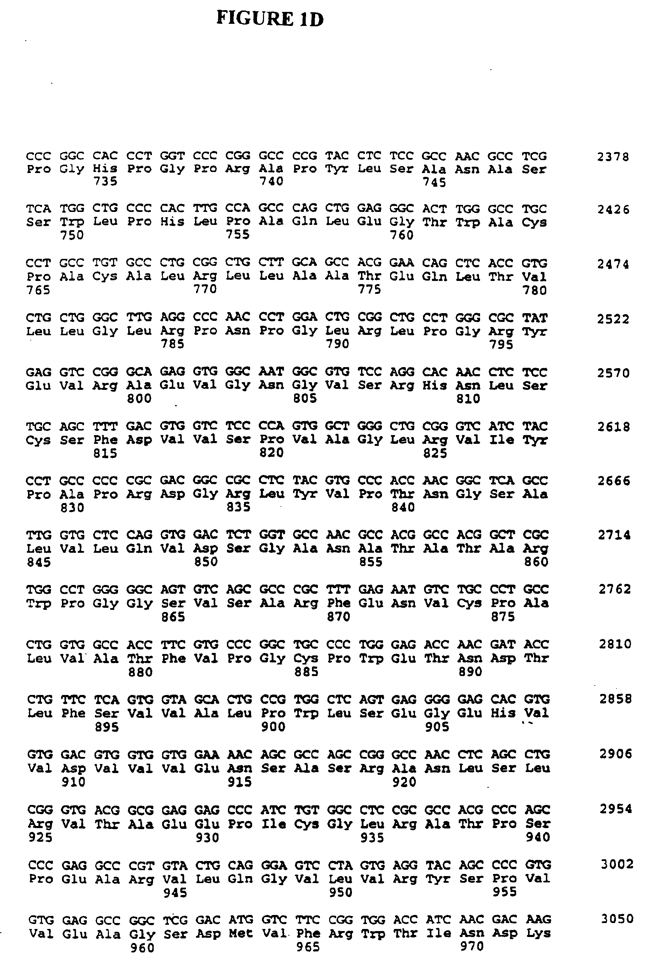

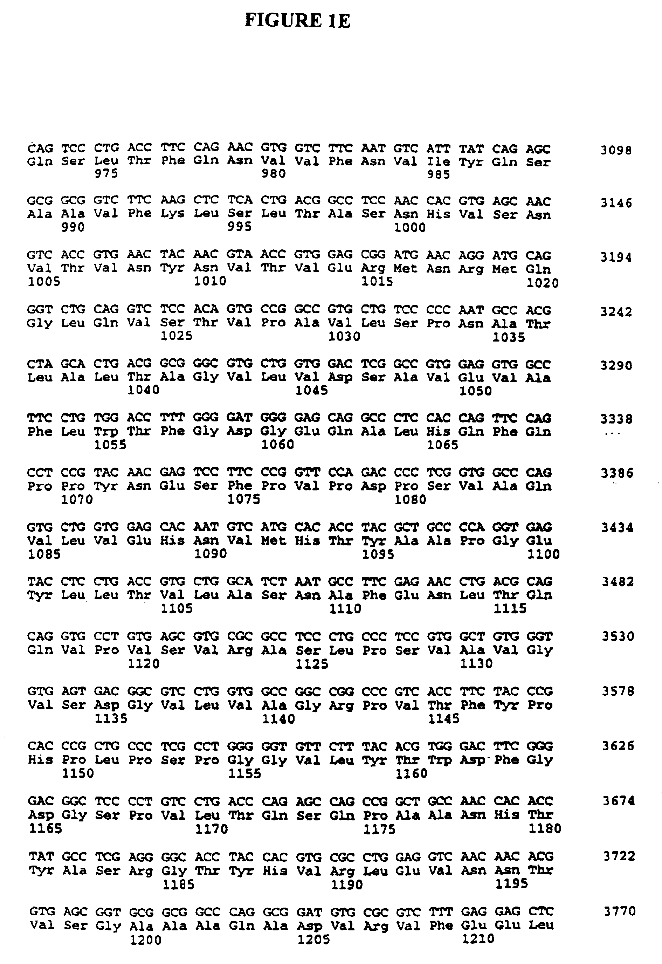

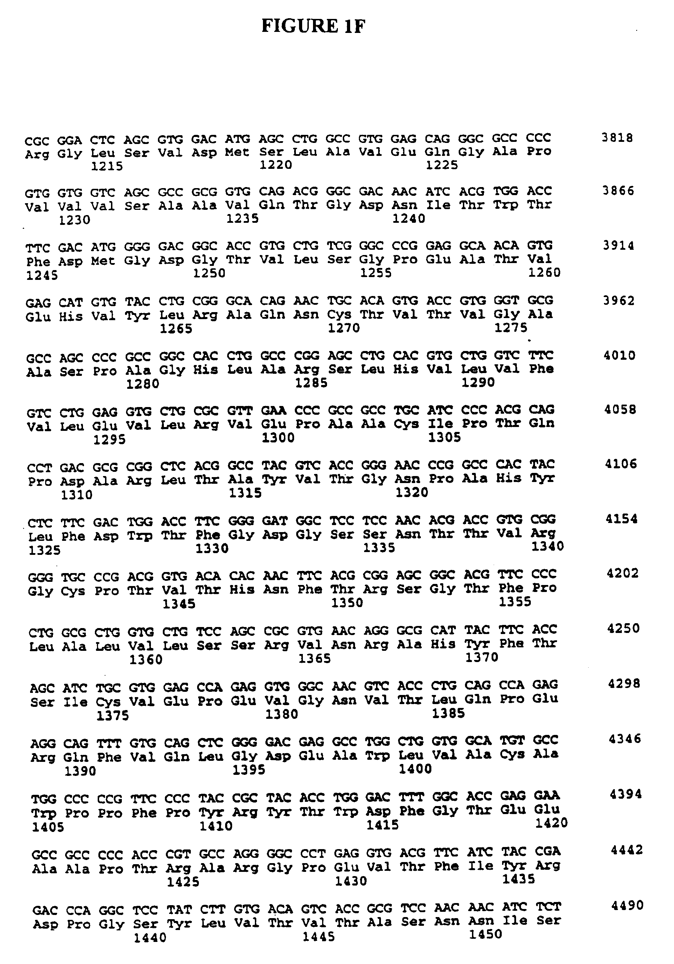

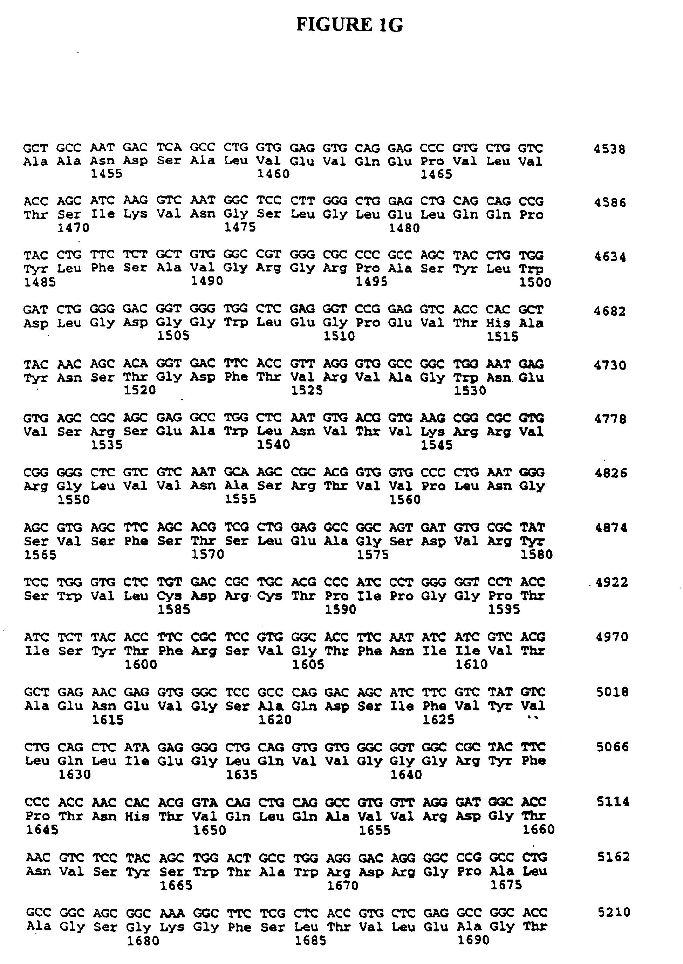

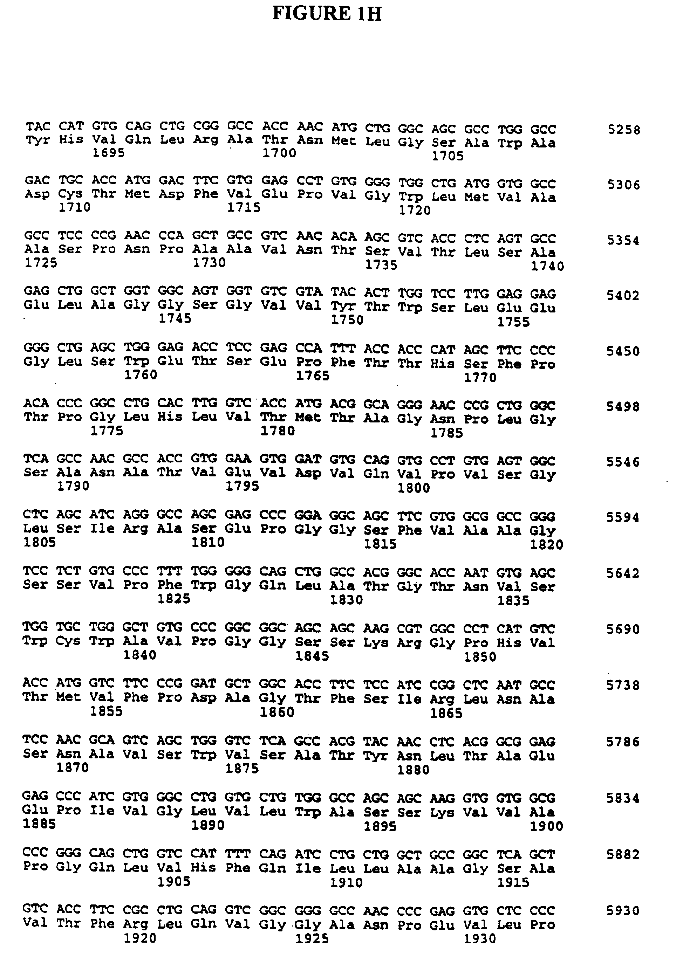

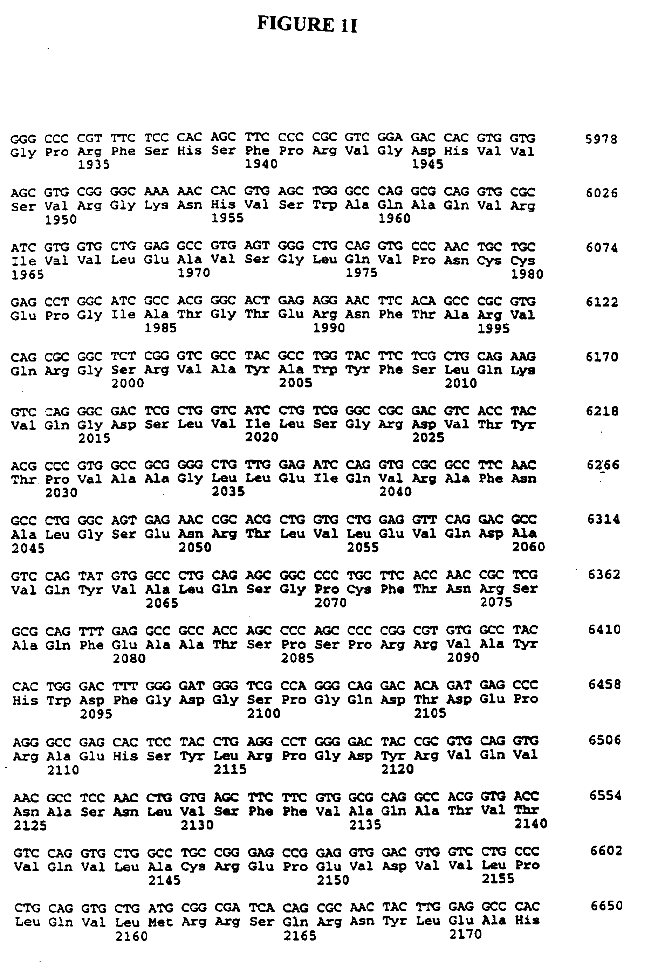

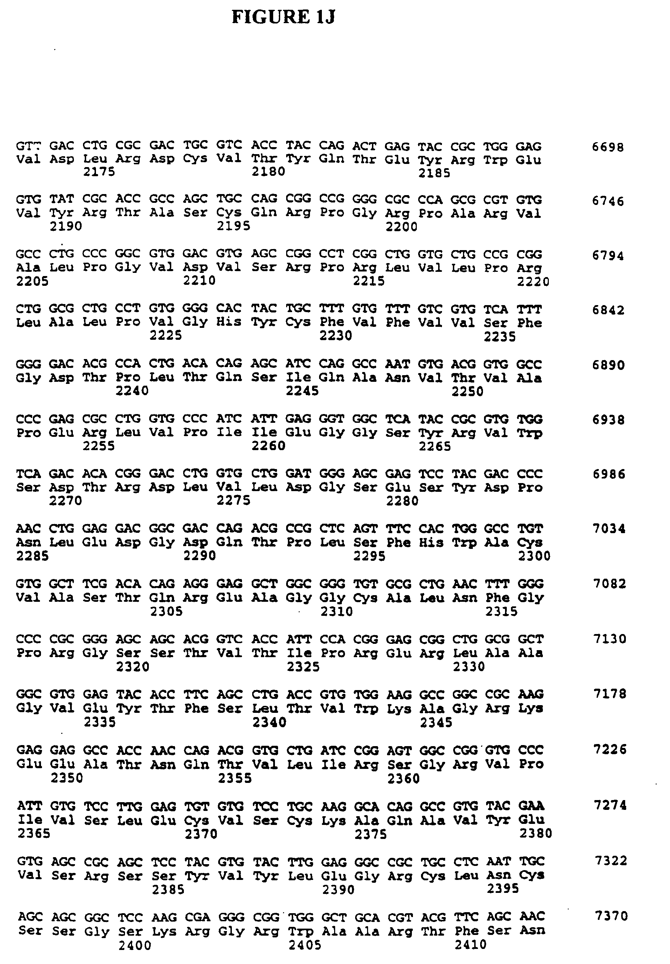

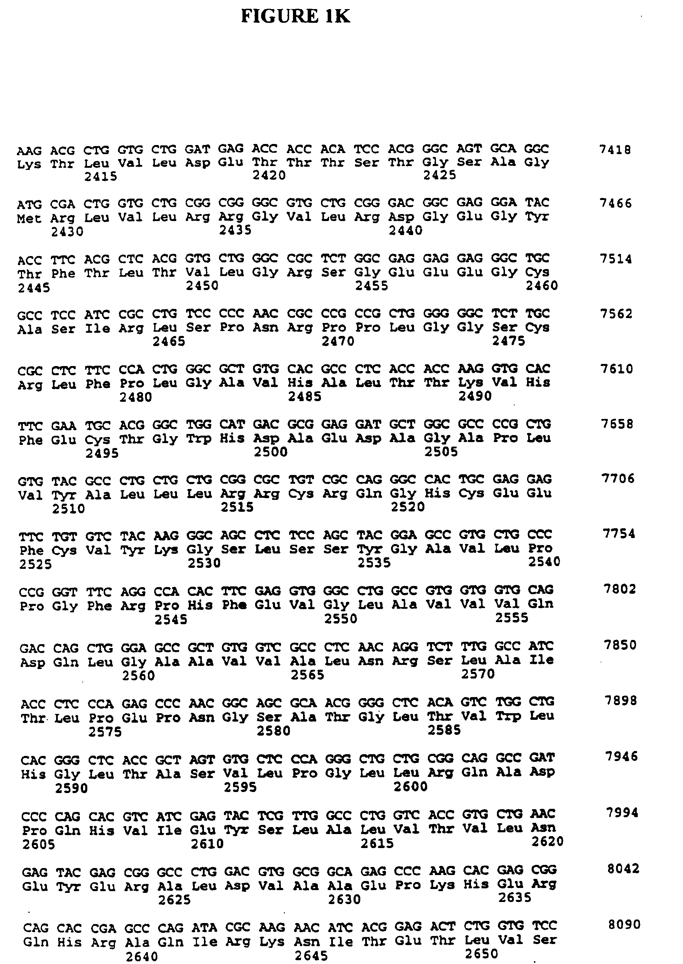

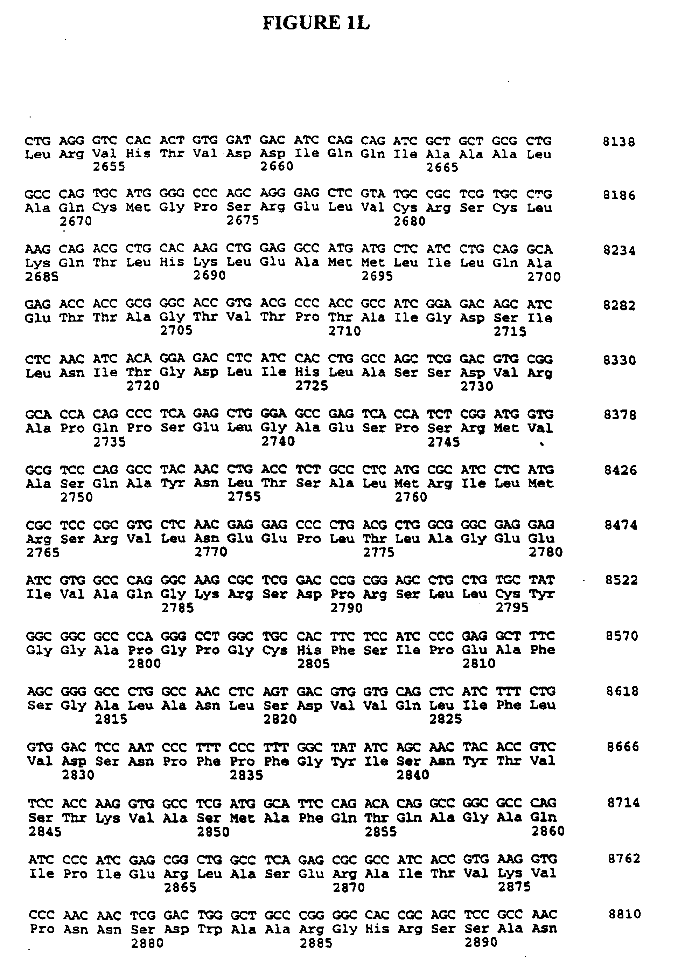

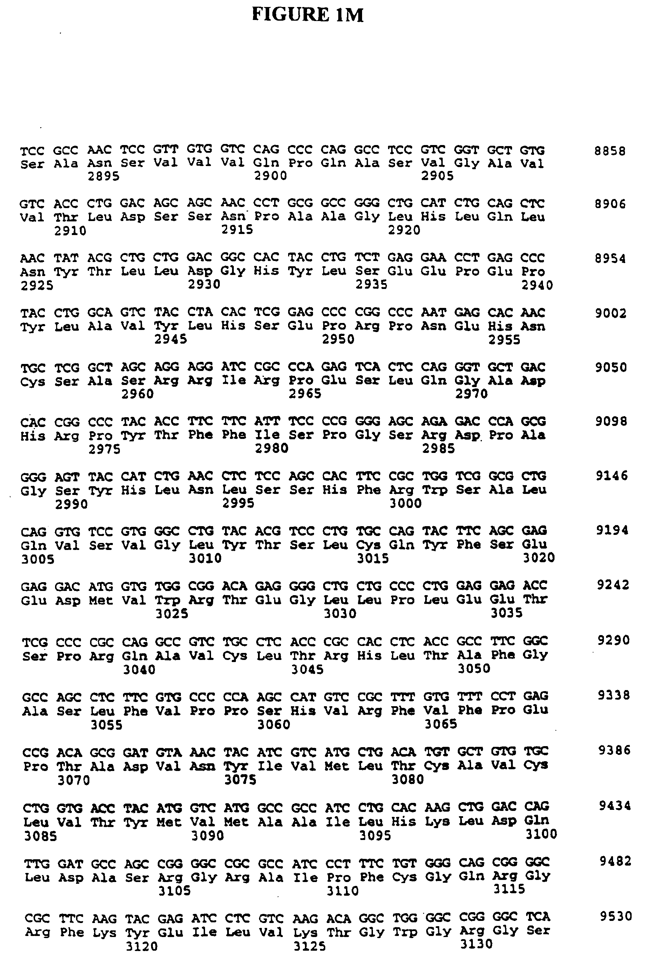

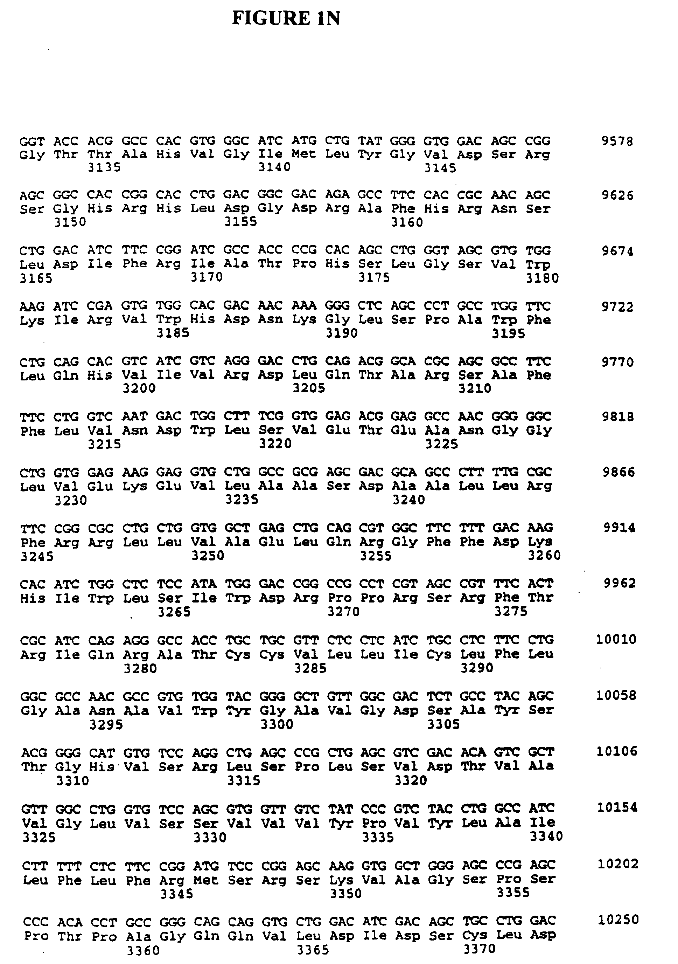

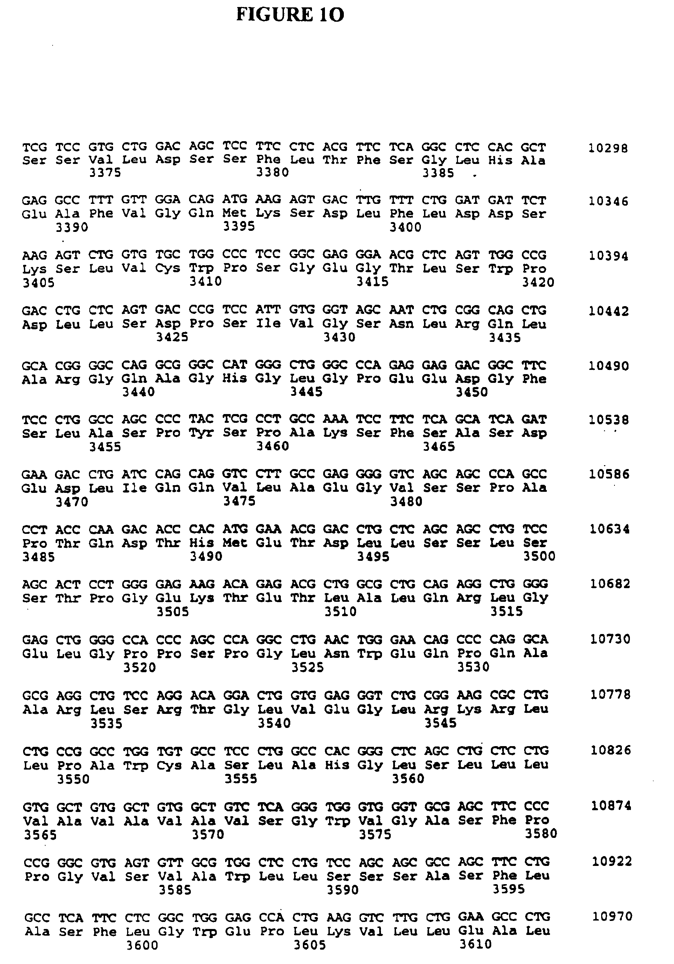

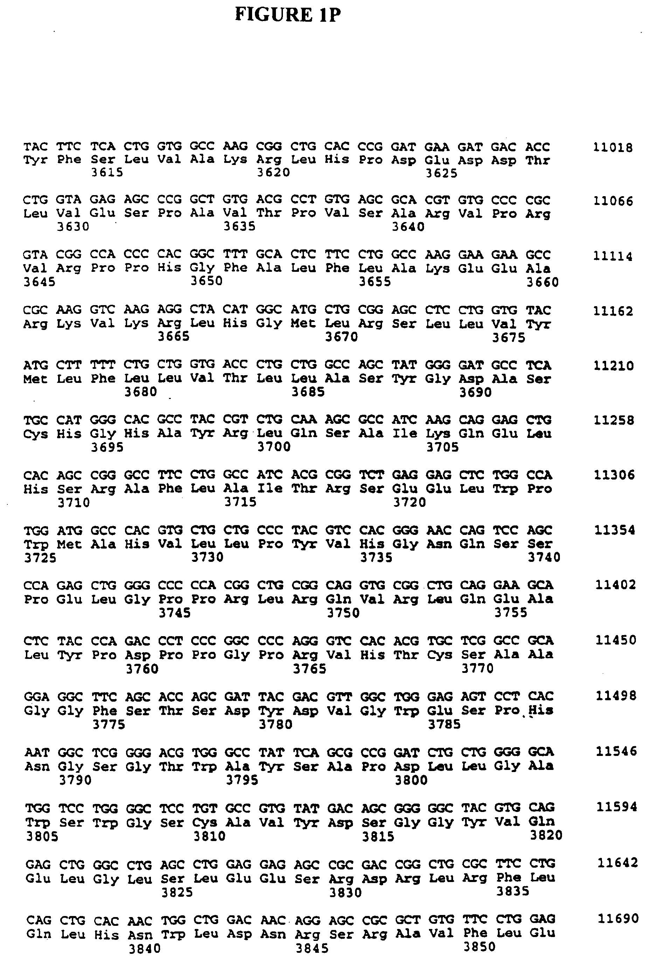

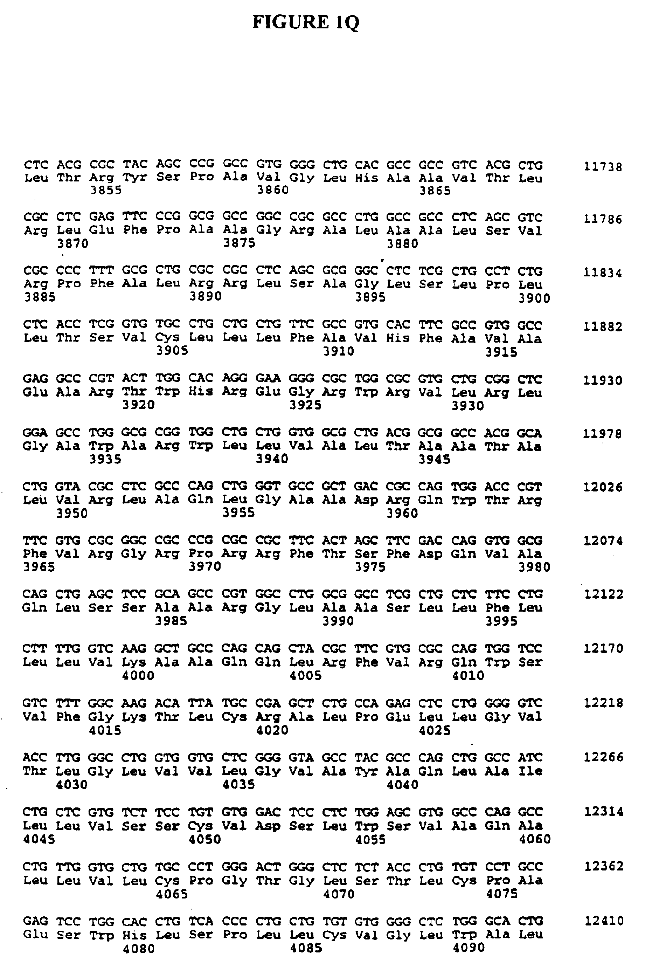

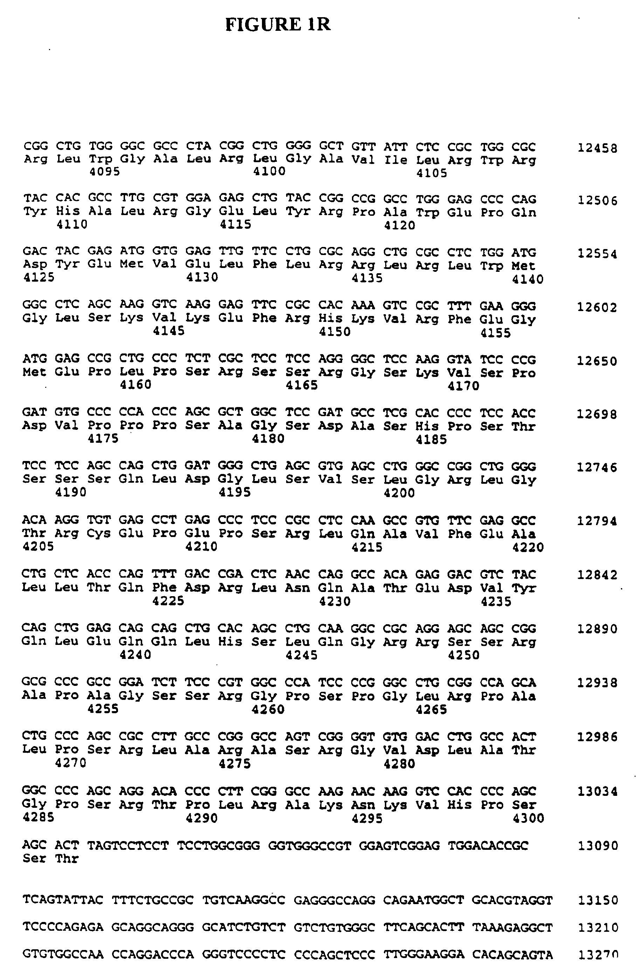

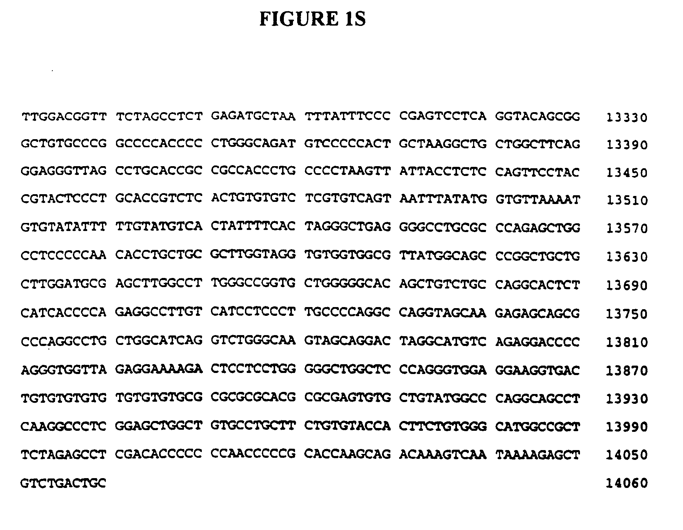

In a further aspect, the invention provides antibodies raised against the Ig-like domains of polycystin, and in particular, peptides comprising amino acids 843 to 1200 or 1205 to 1625 or 1626 to 2136, as shown in Figure 1 (SEQ ID NO:2).

In yet another aspect, the invention provides a hybridoma cell line that produces the monoclonal antibodies of the present invention.

In yet another aspect, the invention provides an isolated polypeptide (PRP) having an apparent molecular weight in the range of about 600 to about 800 kD that specifically binds to an antibody or a fragment thereof as described above.

In still another aspect, the invention provides a recombinant polypeptide comprising a polypeptide fragment of polycystin, yet a further aspect, the polypeptide comprises at least one IgG like domain of polycystin. In still a further aspect, the polypeptide comprises amino acids 843 to 1200 or 1205 to 1625 or 1626 to 2136, as shown in Figure 1 (SEQ ID NO:2).

In still another aspect, the invention provides an isolated polynucleotide encoding the recombinant polypeptide of the present invention.

In other separate aspects, the invention provides an isolated polynucleotide, a gene delivery vehicle, or a cell encoding sequences comprising the polypeptides of the present invention.

An additional aspect of the invention is a method for producing the polypeptides by growing the cells of the invention under conditions favorable for the transcription and translation of the polynucleotide. The polypeptides can be further purified.

A further aspect of the invention also provides methods of generating an antibody or fragment thereof and the methods of using these antibodies for detecting polycystin-related proteins.

In an alternative aspect, the present invention further provides a diagnostic kit for detecting a polycystin-related polypeptide present in a sample, that contains an above-described antibody and instructions for the use of the antibody to detect the polypeptide.

In a yet further aspect, the present invention also provides methods for modulating cell-cell and cell-matrix adhesion in a suitable tissue by delivering to the tissue an effective amount of an agent that modulates the binding of polycystin to its ligand.

In an additional aspect, methods for modulating a pathology associated with disregulation of cell-cell or cell-matrix adhesion in a subject are provided by this invention.

### BRIEF DESCRIPTION OF THE FIGURES

Figure 1 is the polynucleotide sequence of the full-length PKD1 (also referred to herein as "polycystin") cDNA and the predicted amino acid sequence (SEQ ID NOS:1-2).
Figure 2 depicts a panel of 12 fusion proteins comprising the transmembrane sequences of polycystin.

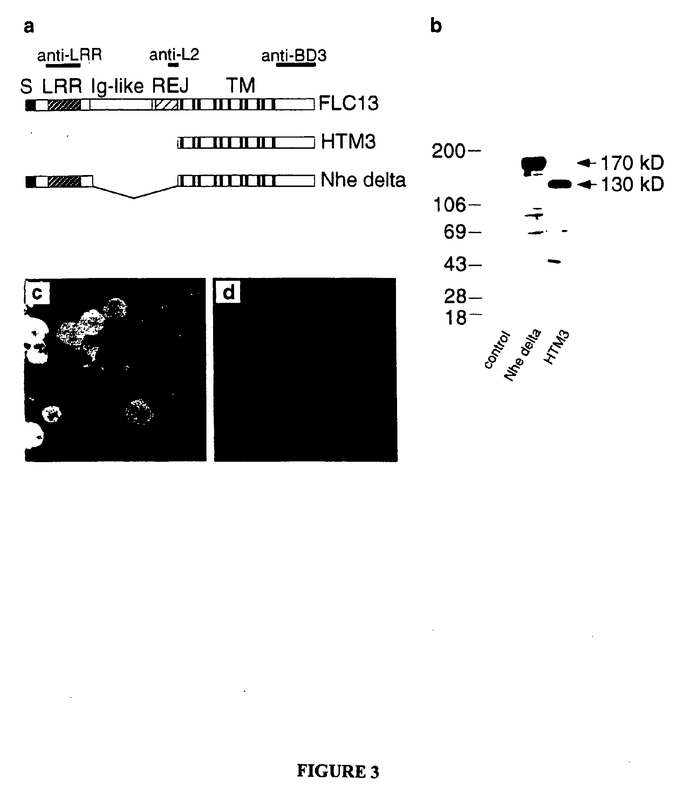

Figure 3A is a schematic representation of the full-length coding region of the PKD1 gene and various deletion constructs of polycystin that were expressed in a baculovirus/insect system and COS cells. The schematic structure of several of expressed recombinant polycystin-1 constructs: FLC13 - full-length polycystin-1 molecule and truncated polycystins - HTM3 (amino acids 3070-4302) and Nhe delta (deletion of amino acids 290 through 2960). Signal peptide (S), leucine rich repeats (LRR) Ig-like repeats (Ig-like), REJ-domain (REJ) and transmembrane regions (TM) are indicated. The epitopes recognized by antibodies are shown by black bars. Figure 3B shows expression of recombinant polycystin-1 and characterization of anti-polycystin-1 antibodies. Immunoblotting of insect Sf21 cells infected with wild-type virus (control), Nhe delta recombinant virus or HTM3 construct (HTM3) with anti-BD3 antibody. Figures 3 C and 3D show immunofluorescence staining using anti-BD3 antibody of Sf21 cells infected with Nhe-delta virus, or with wild-type virus as negative control respectively.

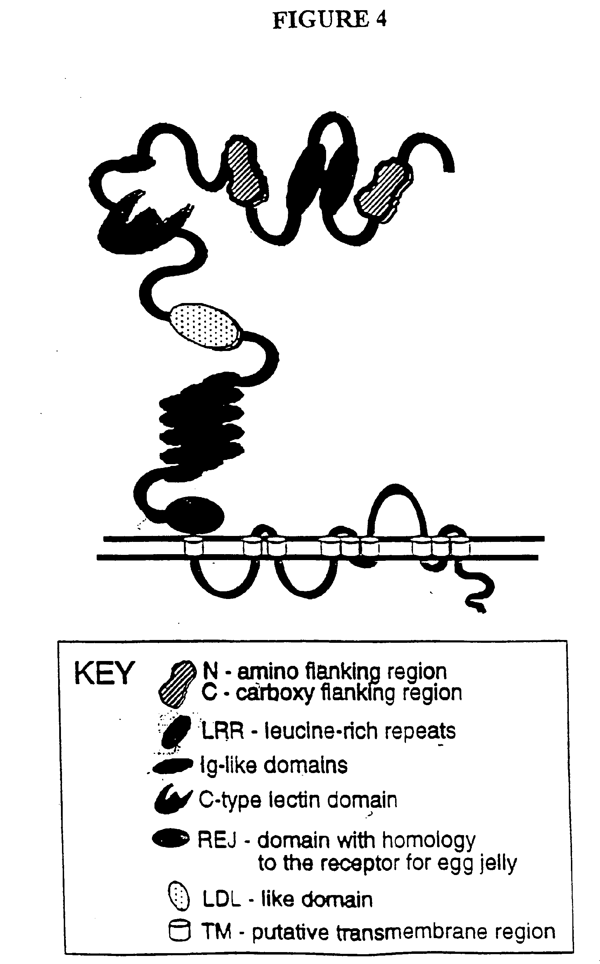

Figure 4 depicts a schematic representation of the full-length coding region of the PKD1 gene with an emphasis on the predicted, conserved domains that are also shared amongst other proteins.

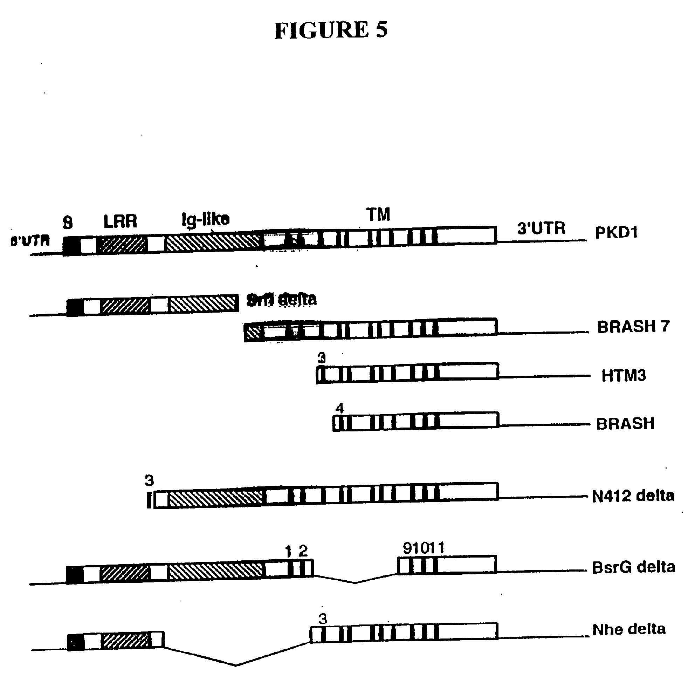

Figure 5 depicts a panel of deletion constructs comprising various domains of polycystin.

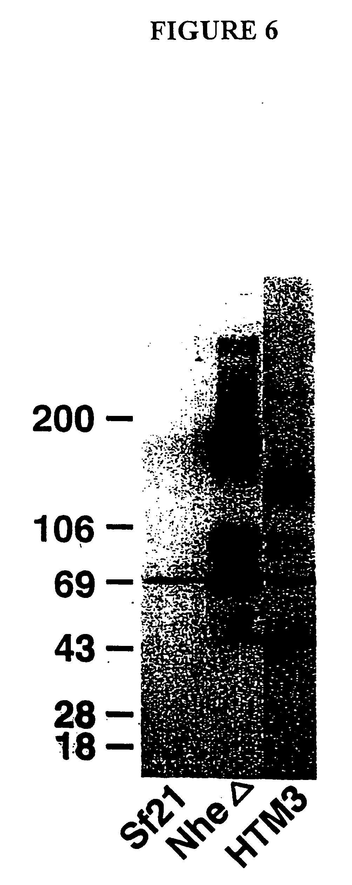

Figure 6 depicts the expression of two truncation mutants of polycystin, Nhe delta and HTM3, in baculovirus/insect system.
Figure 7 depicts an immunoblot demonstrating the detection of the truncated polycystin, Nhe delta, by various antibodies.
Figure 8 depicts the expression of C-terminal part of polycystin in COS 1 cells.
Figure 9 depicts the transient expression of HTM3 in COS1 cells.
Figure 10A depicts the subcellular distribution of a polycystin-related protein in kidney and liver tissues. Figure 10B depicts the differential expression of a polycystin-related protein in microsomal fraction of fetal brain and kidney tissue. Figure 10C depicts the membrane association of a polycystin-related protein in kidney and brain tissues. Figure 10D depicts the expression of a polycystin-related protein in various cell lines.
Figure 11 shows subcellular localization of polycystin-1 in MDCK cells. Immunofluorescence staining with the different anti-polycystin-1 antibodies, anti-LRR, anti-L2 and anti-BD3, each demonstrate intercellular membrane localization of polycystin-1.

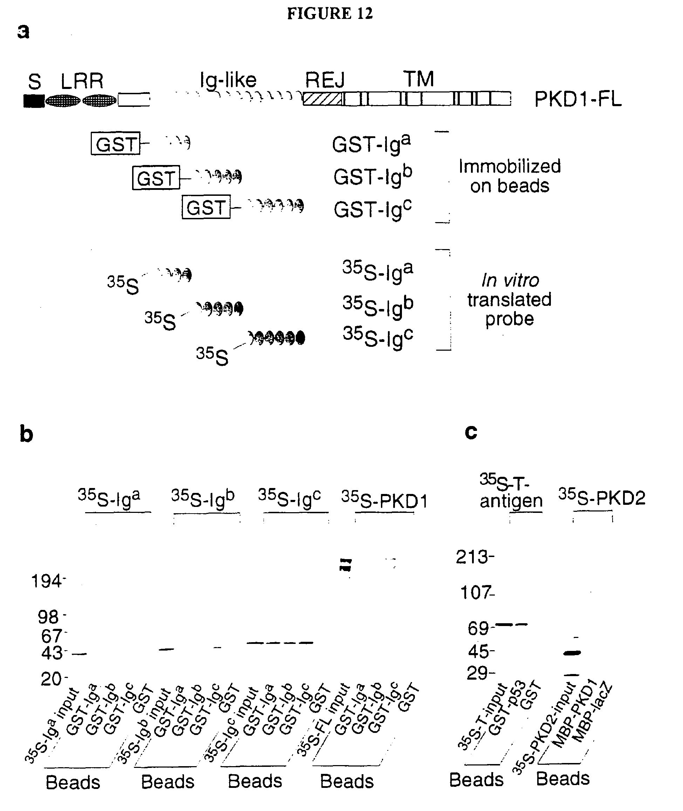

Figure 12 shows *in vitro* binding analysis. In Figure 12A, a schematic structure of the full-length polycystin-1 is indicated with structural motifs. Shown are the fusion protein constructs of Ig-like regions which were immobilized on beads (GST-Ig^{a}, GST-Ig^{b} and GST-Ig^{c}) and the *in vitro* translated probes (³⁵S-Ig^{a}, ³⁵S-Ig^{b}, ³⁵S-Ig^{c}) used for the *in vitro* binding assays. Figure 12B shows homophilic interactions of Ig-like clusters. Autoradiograms *of in vitro* translated ³⁵S-labeled probes of Ig-like regions (shown on top of each panel) specifically bound to bead-immobilized fusion proteins (indicated on the bottom of each panel as GST-Ig^{a}, GST-Ig^{b},GST-Ig^{c} and GST, respectively). The first lane of each panel contains total input of ³⁵S-labeled probe used for each binding experiment. In Figure 12C, the left panel shows an autoradiogram *of in vitro* binding assay for p53 - T-antigen. ³⁵S-T-antigen probe input is shown in lane 1. Lanes 2 and 3 show probe bound to immobilized fusion proteins GST-p53 and GST carrier, respectively. The right panel represents binding of the c-terminal region of the polycystin-2 probe (input shown in the first lane) to immobilized polycystin-1 c-terminal fusion protein (lane 2, MBP-PKD1). Binding of the probe to MBP-lacZ fusion protein was used as negative control (lane 3).

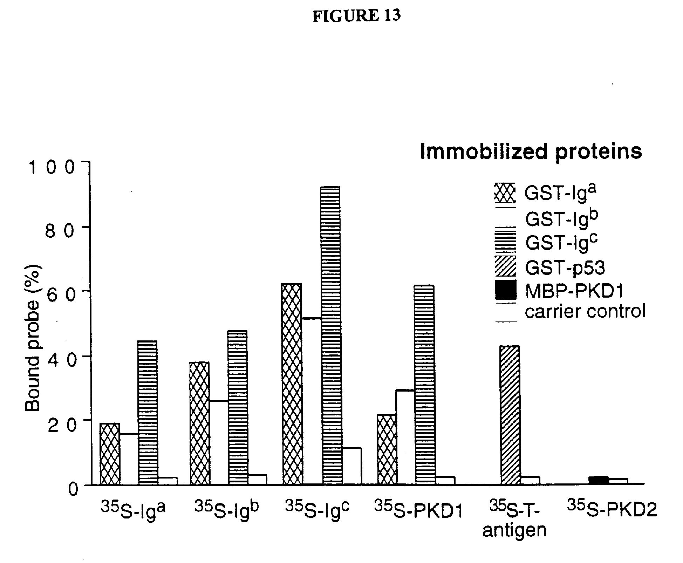

Figure 13 depicts quantitative analysis of Ig-like homophilic interactions. Sepharose beads with immobilized fusion proteins (indicated as immobilized protein) were incubated with ³⁵S-labeled *in vitro* translated probes (shown below). The percentage of bound probe calculated as described in experimental procedures is plotted on the y axis. Beads with corresponding fusion protein carriers (GST or MBP-lacZ) were used as controls for background binding.

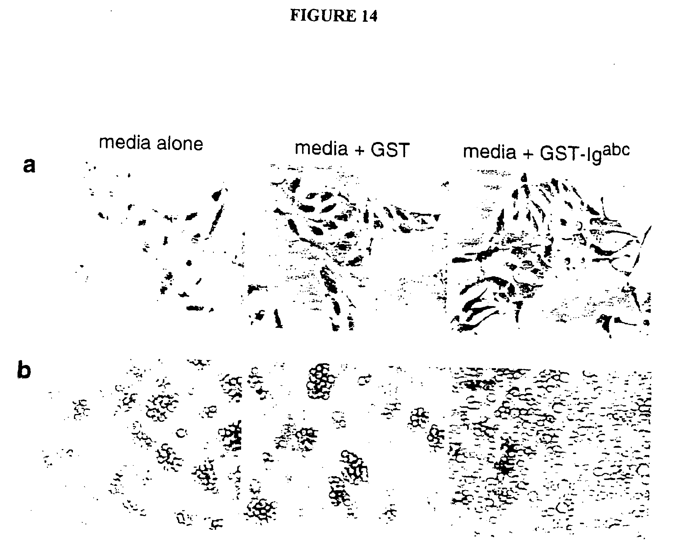

Figure 14 shows the disruption of intercellular adhesion. In Figure 14A, the effect of soluble Ig-like domains of polycystin-1 on cell-cell adhesion in MDCK cell monolayers are shown. Cell monolayers were incubated with GST-Ig^{a}, GST-Ig^{b} and GST-Ig^{c} fusion proteins (media+GST-Ig^{abc}). Note the separation of cells from one another and the fibroblastic morphology of cells at the edge of the island. Cell monolayers incubated with GST protein (media+GST) or grown in the media alone show a compact regularly packed monolayer. Figure 14B shows disruption of aggregate formation by soluble Ig-like domains of polycystin-1. Single MDCK cell suspensions were assayed for their ability to form aggregates in the presence of GST-Ig^{a}, GST-Ig^{b} and GST-Ig^{c} (media+GST-Ig^{ab}). Note the loss of large aggregates in this sample. Formation of large aggregates can be detected easily in the media alone or in the presence of the GST carrier (media+GST) as control.

### MODE(S) FOR CARRYING OUT THE INVENTION

Throughout this disclosure, various publications, patents and published patent specifications are referenced by an identifying citation. The disclosures of these publications, patents and published patent specifications are hereby incorporated by reference into the present disclosure to more fully describe the state of the art to which this invention pertains.

The practice of the present invention will employ, unless otherwise indicated, conventional techniques of immunology, molecular biology, microbiology, cell biology and recombinant DNA, which are within the skill of the art. See, e.g., Sambrook et al. MOLECULAR CLONING: A LABORATORY MANUAL, 2nd edition (1989); CURRENT PROTOCOLS IN MOLECULAR BIOLOGY (F. M. Ausubel et al. eds., (1987)); the series METHODS IN ENZYMOLOGY (Academic Press, Inc.): PCR 2: A PRACTICAL APPROACH (M.J. MacPherson, B.D. Hames and G.R. Taylor eds. (1995)), Harlow and Lane, eds. (1988) ANTIBODIES, A LABORATORY MANUAL, and ANIMAL CELL CULTURE (R.I. Freshney ed. (1987)).

As used in the specification and claims, the singular form "a", "an" and "the" include plural references unless the context clearly dictates otherwise. For example, the term "a cell" includes a plurality of cells, including mixtures thereof.

The term "comprising" is intended to mean that the compositions and methods include the recited elements, but not excluding others. "Consisting essentially of when used to define compositions and methods, shall mean excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. "Consisting of" shall mean excluding more than trace elements of other ingredients and substantial method steps for administering the compositions of this invention. Embodiments defined by each of these transition terms are within the scope of this invention.

The term "polynucleotide" refers to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: a gene or gene fragment, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

A "gene" refers to a polynucleotide containing at least one open reading frame that is capable of encoding a particular protein after being transcribed and translated.

The terms "polypeptide, " "peptide" and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified; for example, disulfide bond formation, glycosylation, lipidation, acetylation, phosphorylation, or any other manipulation, such as conjugation with a labeling component. As used herein the term "amino acid" refers to either natural and/or unnatural or synthetic amino acids, including glycine and both the D or L optical isomers, and amino acid analogs and peptidomimetics.

A protein is associated with polycystic kidney disease when it is present at a substantially altered level or in a substantially altered form in the cells derived from a PDK-affected tissue compared with cells of a control tissue. Such protein may also play a role in renal cystogenesis.

An "integral membrane protein" is a transmembrane protein that extends across the lipid bilayer of the plasma membrane of a cell. A typical integral membrane protein consists of at least one "membrane spanning segment" that generally comprises hydrophobic amino acid residues. Unlike peripheral membrane proteins that can be released from the membrane by relatively gentle extraction procedures, such as exposure to solutions of very high or low ionic strength or extreme pH, integral membrane protein may be linked to the phosphatidylinositols of the bilayer, or be held in the bilayer by a fatty acid chain, and thus can be released only by disrupting the lipid bilayer with detergents or organic solvents. As used herein, "membrane associated" polypeptides include peripheral and integral membrane polypeptides that are bound to any cellular membranes including plasma membranes and membranes of intracellular organelles.

As used herein, the term "antibody" refers to a polypeptide or group of polypeptides which are comprised of at least one antibody combining site. An "antibody combining site" or "binding domain" is formed from the folding of variable domains of an antibody molecule(s) to form three-dimensional binding spaces with an internal surface shape and charge distribution complementary to the features of an epitope of an antigen, which allows an immunological reaction with the antigen. An antibody combining site may be formed from a heavy and/or a light chain domain (VH and VL, respectively), which form hypervariable loops which contribute to antigen binding. The term "antibody" includes, for example, vertebrate antibodies, hybrid antibodies, chimeric antibodies, altered antibodies, univalent antibodies, the Fab proteins, and single domain antibodies. An antibody "specifically binds to" or "specifically recognizes" a polypeptide if it binds with greater affinity or avidity than it binds to other reference polypeptides or substances.

"Antigen" as used herein means a substance that is recognized and bound specifically by an antibody or by a T cell antigen receptor. Antigens can include peptides, proteins, glycoproteins, polysaccharides and lipids; portions thereof and combinations thereof. The antigens can be those found in nature or can be synthetic.

As used herein, the term "epitope" is meant to include any antigenic determinant having specific affinity for the antibodies of the invention. Epitopic determinants usually consist of chemically active surface groupings of molecules such as amino acids or sugar side chains and usually have specific three dimensional structural characteristics, as well as specific charge characteristics. Whereas an epitope can comprise 3 amino acids in a spatial conformation which is unique to the epitope, it generally consists of at least 5 such amino acids, and more usually, consists of at least 8-10 such amino acids. Methods of determining the spatial conformation of amino acids are known in the art, and include, for example, x-ray crystallography and 2-dimensional nuclear magnetic resonance. "Immunological reactivity" as applied to a polypeptide refers to the ability of the polypeptide to specifically bind to an antibody of the present invention. It also refers to the ability of the polypeptide to elicit a specific immune response resulting in the production of antibodies of the present invention.

As used herein, the term "isolated" means separated from constituents, cellular and otherwise, in which the polynucleotide, peptide, polypeptide, protein, antibody, or fragments thereof, are normally associated with in nature. As is apparent to those of skill in the art, a non-naturally occurring the polynucleotide, peptide, polypeptide, protein, antibody, or fragments thereof, does not require "isolation" to distinguish it from its naturally occurring counterpart. In addition, a "concentrated", "separated" or "diluted" polynucleotide, peptide, polypeptide, protein, antibody, or fragments thereof, is distinguishable from its naturally occurring counterpart in that the concentration or number of molecules per volume is greater than "concentrated" or less than "separated" than that of its naturally occurring counterpart.

Enrichment can be measured on an absolute basis, such as weight per volume of solution, or it can be measured in relation to a second, potentially interfering substance present in the source mixture. Increasing enrichments of the embodiments of this invention are increasingly more preferred. Thus, for example, a 2-fold enrichment is preferred, 10-fold enrichment is more preferred, 100-fold enrichment is more preferred, 1000-fold enrichment is even more preferred. A substance can also be provided in an isolated state by a process of artificial assembly, such as by chemical synthesis or recombinant expression.

The "expression" refers to the process by which polynucleotides are transcribed into mRNA and/or the process by which the transcribed mRNA (also referred to as "transcript") is subsequently being translated into peptides, polypeptides, or proteins. The transcripts and the encoded polypeptides are collectedly referred to as gene product. If the polynucleotide is derived from genomic DNA, expression may include splicing of the mRNA in a eukaryotic cell.

"Differentially expressed", as applied to nucleotide sequence or polypeptide sequence in a cell or a tissue, refers to over-expression or underexpression of that sequence when compared to that detected in a control cell or tissue. Underexpression also encompass absence of expression of a particular sequence as evidenced by the absence of detectable expression in a tested sample when compared to a control.

The term "PKD-associated gene" refers to any gene which is yielding transcription or translation products at a substantially altered level or in a substantially altered form in cells derived from PDK-affected tissues compared with cells of a control tissue, and which may play a role in renal cystogenesis. It may be a normally quiescent gene that becomes activated (such as a dominant cyst-causing gene); it may be a gene that becomes expressed at an abnormally high; it may be a gene that becomes mutated to produce a variant phenotype; it may be a gene that becomes expressed at an abnormally low level (such as a cyst suppresser gene); or it may be a gene exhibiting differential expression, in which the differential expression correlates with cyst formation or growth.

The term "hybridize" as applied to a transcript refers to the ability of the transcript to form a complex that is stabilized via hydrogen bonding between the bases of the nucleotide residues in a hybridization reaction. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. The hybridization reaction may constitute a step in a more extensive process, such as the initiation of a PCR reaction, or the enzymatic cleavage of a polynucleotide by a ribozyme.

A "gene delivery vehicle" is defined as any molecule that can carry inserted polynucleotides into a host cell. Examples of gene delivery vehicles are liposomes, viruses, such as baculovirus, adenovirus and retrovirus, bacteriophage, cosmid, plasmid, fungal vectors and other recombination vehicles typically used in the art which have been described for expression in a variety of eukaryotic and prokaryotic hosts, and may be used for gene therapy as well as for simple protein expression.

A "viral vector" is defined as a recombinantly produced virus or viral particle that comprises a polynucleotide to be delivered into a host cell, either in *vivo,* ex vivo *or in vitro.* Examples of viral vectors include retroviral vectors, adenovirus vectors, adeno-associated virus vectors and the like. In aspects where gene transfer is mediated by a retroviral vector, a vector construct refers to the polynucleotide comprising the retroviral genome or part thereof, and a therapeutic gene. As used herein, "retroviral mediated gene transfer" or "retroviral transduction" carries the same meaning and refers to the process by which a gene or nucleic acid sequences are stably transferred into the host cell by virtue of the virus entering the cell and integrating its genome into the host cell genome. The virus can enter the host cell via its normal mechanism of infection or be modified such that it binds to a different host cell surface receptor or ligand to enter the cell. As used herein, retroviral vector refers to a viral particle capable of introducing exogenous nucleic acid into a cell through a viral or viral-like entry mechanism.

Retroviruses carry their genetic information in the form of RNA; however, once the virus infects a cell, the RNA is reverse-transcribed into the DNA form which integrates into the genomic DNA of the infected cell. The integrated DNA form is called a provirus.

In aspects where gene transfer is mediated by a DNA viral vector, such as an adenovirus (Ad) or adeno-associated virus (AAV), a vector construct refers to the polynucleotide comprising the viral genome or part thereof, and a therapeutic gene. Adenoviruses (Ads) are a relatively well characterized, homogenous group of viruses, including over 50 serotypes. (see, e.g., WO 95/27071) Ads are easy to grow and do not require integration into the host cell genome. Recombinant Adderived vectors, particularly those that reduce the potential for recombination and generation of wild-type virus, have also been constructed. (see, WO 95/00655; WO 95/11984). Wild-type AAV has high infectivity and specificity integrating into the host cells genome. (Hermonat and Muzyczka (1984) Proc. Natl. Acad. Sci. USA 81:6466-6470; Lebkowski et al. (1988) Mol. Cell. Biol. 8:3988-3996).

Gene delivery vehicles also include several non-viral vectors, including DNA/liposome complexes, and targeted viral protein DNA complexes. Liposomes that also comprise a targeting antibody or fragment thereof can be used in the methods of this invention. To enhance delivery to a cell, the nucleic acid or proteins of this invention can be conjugated to antibodies or binding fragments thereof which bind cell surface antigens, e.g., TCR, CD3 or CD4.

A "subject," "individual" or "patient" is used interchangeably herein, which refers to a vertebrate, preferably a mammal, more preferably a human. Mammals include, but are not limited to, rabbits, murines, simians, humans, farm animals, sport animals, and pets.

A "control" is an alternative subject or sample used in an experiment for comparison purpose. A control can be "positive" or "negative." For example, where the purpose of the experiment is to detect a differentially expressed transcript or polypeptide in cell or tissue affected by a disease of concern, it is generally preferable to use a positive control (a subject or a sample from a subject, exhibiting such differential expression and syndromes characteristic of that disease), and a negative control (a subject or a sample from a subject lacking the differential expression and clinical syndrome of that disease).

The term "modulate" shall mean upregulate or downregulate as compared to a control response or wild-type response.

### Antibodies and their preparation

An aspect of the present invention is the generation of an antibody capable of binding to the transmembrane domain of polycystin and which specifically recognizes at least one polycystin-related polypeptide having ar apparent molecular weight of about 600 or about 800 kD. Unlike previously characterized antibodies that bind to a PKDI polypeptide(s) of approximately 465 kD, which is consistent with the calculated molecular weight ofpolycystin, the antibodies of the instant invention specifically recognize an endogenous polycystin-related polypeptide having a much higher molecular weight. Such polypeptide has not been previously identified. The polypeptide is expressed in a variety of adult and fetal tissues including but not limited to kidney, liver, brain and neuronal tissues. Also within the scope of the invention are functionally equivalent epitopes, e.g. epitopes having substantially identical amino acid sequences but for a conservative amino acid substitution. Antibodies raised against these functionally equivalent epitopes are also provided in the invention.

Also within the scope of the invention are functionally equivalent epitopes, e.g. epitopes having substantially identical amino acid sequences but for a conservative amino acid substitution. Antibodies raised against these functionally equivalent epitopes are also provided in the invention.

Further encompassed by this invention are antibodies raised against the Ig-like domains of polycystin. Examples of such antibodies include, but are not limited to antibodies raised against peptides comprising amino acids 843 to 1200 or 1205 to 1625 or 1626 to 2136, as shown in Figure 1 (SEQ ID NO:2). Also within the scope of the invention are functionally equivalent epitopes, e.g. epitopes having substantially identical amino acid sequences tut for a conservative amino acid substitution. Antibodies raised against these functionally equivalent epitopes are also provided in the invention.

The antibodies of the present invention encompass polyclonal antibodies and monoclonal antibodies. They include but are not limited to mouse, rat, and rabbit or human antibodies. This invention also encompasses functionally equivalent antibodies and fragments thereof. As used herein with respect to the exemplified antibodies, the phrase "functional equivalent" means an antibody or fragment thereof, or any molecule having the antigen binding site (or epitope) of the antibody that cross-blocks an exemplified antibody when used in an immunoassay such as immunoblotting or immunoprecipitation.

Antibody fragments include the Fab, Fab', F(ab')₂, and Fv regions, or derivatives or combinations thereof. Fab, Fab', and F(ab')₂ regions of an immunoglobulin may be generated by enzymatic digestion of the monoclonal antibodies using techniques well known to those skilled in the art. Fab fragments may be generated by digesting the monoclonal antibody with papain and contacting the digest with a reducing agent to reductively cleave disulfide bonds. Fab' fragments may be obtained by digesting the antibody with pepsin and reductive cleavage of the fragment so produce with a reducing agent. In the absence of reductive cleavage, enzymatic digestion of the monoclonal with pepsin produces F(ab')₂ fragments.

It will further be appreciated that encompassed within the definition of antibody fragment is single chain antibody that can be generated as described in U.S. Pat. No. 4,704,692, as well as chimeric antibodies and humanized antibodies (Oi et al. (1986) BioTechniques 4(3):214). Chimeric antibodies are those in which the various domains of the antibodies' heavy and light chains are coded for by DNA from more than one species.

As used herein with regard to the monoclonal antibody, the "hybridoma cell line" is intended to include all derivatives, progeny cells of the parent hybridoma that produce the monoclonal antibodies specific for the polycystin related proteins, regardless of generation of karyotypic identity.

Laboratory methods for producing polyclonal antibodies and monoclonal antibodies, as well as deducing their corresponding nucleic acid sequences, are known in the art, see Harlow and Lane (1988) *supra* and Sambrook et al. (1989) *supra.* For production of polyclonal antibodies, an appropriate host animal is selected, typically a mouse or rabbit. The substantially purified antigen, whether the whole transmembrane domain, a fragment thereof, or a polypeptide corresponding to a segment of or the entire specific loop region within the transmembrane domain, coupled or fused to another polypeptide, is presented to the immune system of the host by methods appropriate for the host. The antigen is introduced commonly by injection into the host footpads, via intramuscular, intraperitoneal, or intradermal routes. Peptide fragments suitable for raising antibodies may be prepared by chemical synthesis, and are commonly coupled to a carrier molecule (e.g., keyhole limpet hemocyanin) and injected into a host over a period of time suitable for the production of antibodies. Alternatively, the antigen can be generated recombinantly as a fusion protein. Examples of components for these fusion proteins include, but are not limited to myc, HA, FLAG, His-6, glutathione S-transferase, maltose binding protein or the Fc portion of immunoglobulin.

The monoclonal antibodies of this invention refer to antibody compositions having a homogeneous antibody population. It is not intended to be limited as regards to the source of the antibody or the manner in which it is made. Generally, monoclonal antibodies are biologically produced by introducing protein or a fragment thereof into a suitable host, e.g., a mouse. After the appropriate period of time, the spleens of such animal is excised and individual spleen cells fused, typically, to immortalized myeloma cells under appropriate selection conditions. Thereafter the cells are clonally separated and the supernatants of each clone are tested for their production of an appropriate antibody specific for the desired region of the antigen using methods well known in the art.

The isolation of other hybridomas secreting monoclonal antibodies with the specificity of the monoclonal antibodies of the invention can also be accomplished by one of ordinary skill in the art by producing anti-idiotypic antibodies (Herlyn et al. (1986) Science 232:100). An anti-idiotypic antibody is an antibody which recognizes unique determinants present on the monoclonal antibody produced by the hybridoma of interest.

Idiotypic identity between monoclonal antibodies of two hybridomas demonstrates that the two monoclonal antibodies are the same with respect to their recognition of the same epitopic determinant. Thus, by using antibodies to the epitopic determinants on a monoclonal antibody it is possible to identify other hybridomas expressing monoclonal antibodies of the same epitopic specificity.

It is also possible to use the anti-idiotype technology to produce monoclonal antibodies which mimic an epitope. For example, an anti-idiotypic monoclonal antibody made to a first monoclonal antibody will have a binding domain in the hypervariable region which is the mirror image of the epitope bound by the first monoclonal antibody. Thus, in this instance, the anti-idiotypic monoclonal antibody could be used for immunization for production of these antibodies.

Other suitable techniques of antibody production include, but are not limited to, *in vitro* exposure of lymphocytes to the antigenic polypeptides or selection of libraries of antibodies in phage or similar vectors. See Huse et al. (1989) Science 246:1275-1281. Genetically engineered variants of the antibody can be produced by obtaining a polynucleotide encoding the antibody, and applying the general methods of molecular biology to introduce mutations and translate the variant. The above described antibody "derivatives" are further provided herein.

Sera harvested from the immunized animals provide a source of polyclonal antibodies. Detailed procedures for purifying specific antibody activity from a source material are known within the art. Undesired activity cross-reacting with other antigens, if present, can be removed, for example, by running the preparation over adsorbants made of those antigens attached to a solid phase and eluting or releasing the desired antibodies off the antigens. If desired, the specific antibody activity can be further purified by such techniques as protein A chromatography, ammonium sulfate precipitation, ion exchange chromatography, high-performance liquid chromatography and immunoaffinity chromatography on a column of the immunizing polypeptide coupled to a solid support.

The specificity of an antibody refers to the ability of the antibody to distinguish polypeptides comprising the immunizing epitope from other polypeptides. If an antibody or fragment thereof being tested binds to an epitope in the transmembrane domain of polycystin and recognizes a related protein having a molecular weight of about 600 or about 800 kD, then the antibody being tested and the antibodies provided by this invention have the same specificity. An ordinary skill in the art can readily determine without undue experimentation whether an antibody shares the same specificity as an antibody of this invention by determining whether the antibody being tested prevents an antibody of this invention from binding the polypeptide(s) with which the antibody is normally reactive. If the antibody being tested competes with the antibody of the invention as shown by a decrease in binding by the antibody of this invention, then it is likely that the two antibodies bind to the same or a closely related epitope. Alternatively, one can pre-incubate the antibody of this invention with the polypeptide(s) with which it is normally reactive, and determine if the antibody being tested is inhibited in its ability to bind the antigen. If the antibody being tested is inhibited, then, in all likelihood, it has the same, or a closely related, epitopic specificity as the antibody of this invention.

The antibodies of the invention can be bound to many different carriers. Thus, this invention also provides compositions containing antibodies and a carrier. Carriers can be active and/or inert. Examples of well-known carriers include polypropylene, polystyrene, polyethylene, dextran, nylon, amylases, glass, natural and modified celluloses, polyacrylamides, agaroses and magnetite. The nature of the carrier can be either soluble or insoluble for purposes of the invention. Those skilled in the art will know of other suitable carriers for binding antibodies, or will be able to ascertain such, using routine experimentation.

The antibodies of this invention can also be conjugated to a detectable agent or a hapten. The complex is useful to detect the polypeptide(s) (or polypeptide fragments) to which the antibody specifically binds in a sample, using standard immunochemical techniques such as immunohistochemistry as described by Harlow and Lane (1988), *supra.* There are many different labels and methods of labeling known to those of ordinary skill in the art. Examples of the types of labels which can be used in the present invention include radioisotopes, enzymes, colloidal metals, fluorescent compounds, bioluminescent compounds, and chemiluminescent compounds. Those of ordinary skill in the art will know of other suitable labels for binding to the antibody, or will be able to ascertain such, using routine experimentation. Furthermore, the binding of these labels to the antibody of the invention can be done using standard techniques common to those of ordinary skill in the art.

Another technique which may also result in greater sensitivity consists of coupling the antibodies to low molecular weight haptens. These haptens can then be specifically detected by means of a second reaction. For example, it is common to use such haptens as biotin, which reacts avidin, or dinitrophenyl, pyridoxal, and fluorescein, that can react with specific anti-hapten antibodies. See Harlow and Lane (1988), *supra.*

### Polypeptides of the present invention

This present invention encompasses polypeptides separately comprising the transmembrane and Ig-like domains of a PKD1 gene product. These transmembrane domain specific polypeptides are characterized by their ability to elicit a humoral and/or cellular immune response in a host that results in production of antibodies capable of detecting novel polypeptides related to the polycystin protein family. The antibodies bind to the Ig-like domains of polycystin and block binding of polycystin to its ligand. These antibodies also useful to modulate cell-cell and cell-tissue adhesion in a suitable tissue.

The polypeptides of this invention also comprise fragments of the PKD protein comprising the Ig-like domains. In one embodiment, the polypeptide comprises regions II-V (Figure 1, amino acids 843 to 1200). In a separate embodiment, the polypeptide comprises regions VI to X (Figure 1, amino acids 1205 to 1625). In a further embodiments, the polypeptide comprises regions XI to XVI (Figure 1, amino acids 1626 to 2136). The Ig-like polypeptides of this invention are useful to enhance or promote cell-cell or cell-matrix adhesion in a suitable tissue because they are shown to mediate interactions between these domains. In some situations, where due to mutation, a soluble form of extracellular domains, including Ig-like domains, can be produced. The soluble proteins can disrupt the cell-cell adhesion. The antibodies of this invention are useful to bind and/or remove the soluble, mutated polycystin thereby restoring normal adhesion to tissue. The antibodies are further useful in screens to identify agents that may prevent or treat pathologies related to the disregulation of the PKD gene in a subject as described above.

Such tissue includes, but is not limited to kidney, brain liver or neuronal. Additional suitable tissues can be screened using the antibodies that specifically recognize and bind the loop domains. If the antibody binds to the tissue, the tissue expresses polycystin.

This invention also provides a novel polypeptide that differs from the previously characterized polycystin polypeptides in that they contain additional amino acid sequences and/or post-translationally modified motifs, and exhibit a mobility on a SDS-PAGE gel of about 600 kD or about 800 kD, that are approximately 200 to 400 kD higher than that predicted for polycystin.

The predicted amino acid sequence of full-length polycystin is shown in Figure 1 (SEQ ID NO:2).

In yet another embodiment, the present invention provides an isolated polypeptide having an apparent molecular weight of about 600 or about 800 kD, which specifically binds to an above-described antibody or fragment thereof, The polypeptide exhibits sequence homology with polycystin, as it binds to the antibodies raised against epitopes present in the transmembrane domain of polycystin. It can be isolated from cellular constituents with which it is normally associated by conventional protein purification techniques. Non limiting examples include ammonium sulfate precipitation, gel electrophoresis, ion exchange chromatography, and high-performance liquid chromatography. A preferred method is immunoaffinity chromatography using antibodies to which the polypeptide binds. Where desired, the amino acid sequences of the 600 kD and 800 kD protein and fragments thereof can be determined by methods well established in the art.

In one embodiment, the polypeptide is expressed in a tissue selected from the group consisting of kidney, brain, liver and neuronal tissues In another embodiment, the polypeptide is associated with cellular membranes including the plasma membrane and membranes of cellular organelles. Non limiting examples of cellular organelles include Golgi, endoplasmic reticulum, lysosome, and mitochondria. In yet another embodiment, the polypeptide is an integral membrane protein. In still another embodiment, the polypeptide is a cytosolic protein (i.e., distributed predominantly or about equally in the membrane and cytosolic fractions). Such polypeptide may be an isoform of polycystin that is unprocessed, variably spliced, or differentially expressed in cells or tissues, such as those affected by polycystic kidney disease. The polypeptide may also be a mutated variant that is involved in pathogenic events leading to kidney cyst formation.

It is understood that biological or functional equivalents or derivatives of the exemplified polypeptides are also encompassed by this invention. A "functionally equivalent" varies from the native sequence disclosed herein by any combination of additions, deletions, or substitutions while preserving at least one functional property of the fragment relevant to the context in which it is being used. A functional equivalent of a polypeptide of the invention typically has the ability to elicit an immune response with a similar antigen specificity as that elicited by exemplified polypeptides or to mediate cell-cell or cell-matrix adhesion. For example, the size of the polypeptide fragments useful for immunizing a host may vary widely, as the length required to affect an immune response could be as small as, for example, a 3-mer amino acid sequence. The maximum length generally is not detrimental to effecting activity. The minimum size must be sufficient to provide a desired function. Thus, the invention includes polypeptide fragments comprising a portion of the transmembrane amino acid sequences exemplified herein, in which the polypeptide is at least about 3, more preferably about 50, more preferably about 75, more preferably 100, more preferably 200 or more, amino acids in length. As is apparent to one skilled in the art, these polypeptides, regardless of their size, may also be associated with, or conjugated with, other substances or agents to facilitate, enhance, or modulate their function.

The invention includes modified polypeptides containing conservative or non-conservative substitutions that do not significantly affect their properties, such as the immunogenicity of the peptides. Modification of polypeptides is routine practice in the art. Amino acid residues which can be conservatively substituted for one another include but are not limited to: glycine/alanine; valine/isoleucine/leucine; asparagine/glutamine; aspartic acid/glutamic acid; serine/threonine; lysine/arginine; and phenylalanine/tryosine. These polypeptides also include glycosylated and nonglycosylated polypeptides, as well as polypeptides with other post-translational modifications, such as, for example, glycosylation with different sugars, acetylation, and phosphorylation.

The polypeptides of the invention can also be conjugated to a chemically functional moiety. Typically, the moiety is a label capable of producing a detectable signal. These conjugated polypeptides are useful, for example, in detection systems such as imaging of renal cysts. Such labels are known in the art and include, but are not limited to, radioisotopes, enzymes, fluorescent compounds, chemiluminescent compounds, bioluminescent compounds substrate cofactors and inhibitors. See, for examples of patents teaching the use of such labels, U.S. Patent Nos. 3,817,837; 3,850,752; 3,939,350; 3,996,345; 4,277,437; 4,275,149; and 4,366,241. The moieties can be covalently linked to the polypeptides, recombinantly linked, or conjugated to the polypeptides through a secondary reagent, such as a second antibody, protein A, or a biotin-avidin complex.

Other functional moieties include agents that enhance immunological reactivity, agents that facilitate coupling to a solid support, vaccine carriers, bioresponse modifiers, paramagnetic labels and drugs. Agents that enhance immunological reactivity include, but are not limited to, bacterial superantigens. Agents that facilitate coupling to a solid support include, but are not limited to, biotin or avidin. Immunogen carriers include, but are not limited to, any physiologically acceptable buffers.

The invention also encompasses fusion proteins comprising polycystin transmembrane sequences and Ig-like domains and fragments thereof. Such fusion may be between two or more polycystin transmembrane or Ig-like sequences or between the sequences of polycystin and a related or unrelated polypeptide. Useful fusion partners include sequences that enhance immunological reactivity, or facilitate the coupling of the polypeptide to an immunoassay support or a vaccine carrier. For instance, the polycystin transmembrane sequences can be fused with a bioresponse modifier. Examples of bioresponse modifiers include, but are not limited to, cytokines or lymphokines such as interleukin-2 (IL-2), interleukin 4 (IL-4), GM-CSF, and interferon. Another useful fusion sequence is one that facilitates purification. Examples of such sequences are known in the art and include those encoding epitopes such as Myc, HA (derived from influenza virus hemagglutinin), His-6, or FLAG. Other fusion sequences that facilitate purification are derived from proteins such as glutathione S-transferase (GST), maltose-binding protein (MBP), or the Fc portion of immunoglobulin. For immunological purposes, tandemly repeated polypeptide segments may be used as antigens, thereby producing highly immunogenic proteins.

The proteins of this invention also can be combined with various liquid phase carriers, such as sterile or aqueous solutions, pharmaceutically acceptable carriers, suspensions and emulsions. Examples of non-aqueous solvents include propyl ethylene glycol, polyethylene glycol and vegetable oils. When used to prepare antibodies, the carriers also can include an adjuvant that is useful to non-specifically augment a specific immune response. A skilled artisan can easily determine whether an adjuvant is required and select one. However, for the purpose of illustration only, suitable adjuvants include, but are not limited to Freund's Complete and Incomplete, mineral salts and polynucleotides.

The proteins and polypeptides of this invention are obtainable by a number of processes well known to those of skill in the art, which include purification, chemical synthesis and recombinant methods. Full-length proteins can be purified from a cell derived from polycystic tissue or tissue lysate by methods such as immunoprecipitation with antibody, and standard techniques such as gel filtration, ion-exchange, reversed-phase, and affinity chromatography using a fusion protein as shown herein. For such methodology, see for example, Deutscher et al. (1999) GUIDE TO PROTEIN PURIFICATION: METHODS IN ENZYMOLOGY (Vol. 182, Academic Press). Accordingly, this invention also provides the processes for obtaining these proteins and polypeptides as well as the products obtainable and obtained by these processes.

The proteins and polypeptides also can be obtained by chemical synthesis using a commercially available automated peptide synthesizer such as those manufactured by Perkin Elmer/Applied Biosystems, Inc., Model 430A or 431A, Foster City, CA. The synthesized protein or polypeptide can be precipitated and further purified, for example by high performance liquid chromatography (HPLC). Accordingly, this invention also provides a process for chemically synthesizing the proteins of this invention by providing the sequence of the protein and reagents, such as amino acids and enzymes and linking together the amino acids in the proper orientation and linear sequence.

Alternatively, the proteins and polypeptides can be generated recombinantly by expressing polynucleotides using the vector systems and host cells as described in the section that follows.

The polypeptides or proteins embodied in the present invention can be characterized in several ways. For instance, a polycystin-related polypeptide may be tested for its ability to bind specifically to an antibody described herein, or for its ability to specifically interfere the binding between another polypeptide and an antibody of the present invention. The ability of a polypeptide to bind specific antibodies can be tested by immunoassay. In one such assay, the antibody is labeled. Suitable labels include radioisotopes such as ¹²⁵I, enzymes such as peroxidase, fluorescent labels such as fluorescein, and chemiluminescent labels. Typically, the other binding partner is immobilized to a solid phase, e.g., by coating onto a microtiter plate or by coupling to beads. For such solid-phase assay, the unreacted antibodies are removed by washing. In a liquid-phase assay, however, the unreacted antibodies are removed by some other separation technique, such as filtration or chromatography. After binding the polypeptides to the antibodies, the amount of bound label is determined. A variation of this technique is a competitive assay, in which the tested polypeptide is titered for its ability to decrease the binding of antibodies specific for, e.g., the 600 kD or 800 kD polycystin-related protein.

### Polynucleotides, vectors and cells of the present invention

The invention provides various polynucleotides that encode the polypeptides of the invention. The polynucleotides are selected based on the predicted transmembrane and Ig-like domain sequences of the PKD1 gene. The transmembrane polynucleotides yield proteins or polypeptides that elicit, in a suitable host, domain specific antibodies that are capable of binding to a novel polypeptide exhibiting a molecular mobility (approximately 600 kD or 800 kD on a SDS-PAGE gel) distinct from the previously characterized polycystin protein. The Ig-like domain polynucleotides yield proteins or polypeptides that mediate or facilitate cell-cell or cell-matrix adhesion.

In a further embodiment, an isolated polynucleotide encodes a polypeptide corresponding to the Ig-like domains in polycystin-1. Such polypeptides include, but are not limited to polypeptides comprising amino acids 843 to 1200 or 1205 to 1625 or 1626 to 2136, as shown in Figure 1.

It is understood that the polynucleotides embodied in the invention include those coding for biological equivalents and fragments of the exemplified polypeptides. Biologically equivalent polypeptides include those which do not significantly affect properties of the polypeptides encoded thereby. Biological equivalents include, but are not limited to polypeptides having conservative amino acid substitutions, analogs including fusions, and muteins.

While the length of a polynucleotide may vary widely the polynucleotide of the present invention preferably comprises at least 15 consecutive nucleotides, preferably at least about 150 consecutive nucleotides, more preferably at least about 225 consecutive nucleotides, even more preferably at least about 300 consecutive nucleotides, still more preferably at least about 300 consecutive nucleotides, that hybridizes with a polynucleotide encoding a polypeptide comprising sequences of the transmembrane loop region 1, 2, 3, 4, or 7. In an alternative embodiment, the polynucleotides hybridize under moderate or stringent conditions to the polynucleotides that encode a polypeptide comprising amino acids 843 to 1200 or 1205 to 1625 or 1626 to 2136, as shown in Figure 1.

Hybridization can be performed under conditions of different "stringency." Conditions that vary levels of stringency are well known in the art See, for example, Sambrook et al., *supra.* Briefly, relevant conditions include temperature, ionic strength, time of incubation, the presence of additional solutes in the reaction mixture such as formamide, and the washing procedure. Higher stringency conditions are those conditions, such as higher temperature and lower sodiums ion concentration, which require higher minimum complementarity between hybridizing elements for a stable hybridization complex 10 form. In general, a low stringency hybridization reaction is carried out at about 40° C in 10 x SSC or a solution of equivalent ionic strength/temperature. A moderate stringency hybridization is typically performed at about 50° C in 6 X SSC, and a high stringency hybridization reaction is generally performed at about 60° C in 1 X SSC. In choosing a polynucleotide most closely related to those encoding the exemplary polypeptides, stringent hybridization is preferred.

This invention also encompasses "biologically equivalent" polynucleotides that encode polypeptides having the biological activity of wild-type polypeptides, but differ in primary polypeptide or polynucleotide sequences. Biologically equivalent polynucleotides can be identified using sequence homology searches. Several embodiments of biologically equivalent polynucleotides are within the scope of this invention, e.g., those characterized by possessing at least 75%, or at least 80%, or at least 90% or at least 95% sequence homology as determined using a sequence alignment program under default parameters correcting for ambiguities in the sequence data, changes in nucleotide sequence that do not alter the amino acid sequence because of degeneracy of the genetic code, conservative amino acid substitutions and corresponding changes in nucleotide sequence, and variations in the lengths of the aligned sequences due to splicing variants or small deletions or insertions between sequences that do not affect function.

A variety of software programs are available in the art. Non-limiting examples of these programs are BLAST family programs including BLASTN, BLASTP, BLASTX, TBLASTN, and TBLASTX (BLAST is available from the worldwide web at http://www.ncbi.nlm.nih.govBLAST/), FastA, Compare, DotPlot, BestFit, GAP, FrameAlign, ClustalW, and PileUp. These programs can be obtained commercially in a comprehensive package of sequence analysis software such as GCG Inc.'s Wisconsin Package. Other similar analysis and alignment programs can be purchased from various providers such as DNA Star's MegAlign, or the alignment programs in GeneJockey. Alternatively, sequence analysis and alignment programs can be accessed through the world wide web at sites such as the CMS Molecular Biology Resource at http://www.sdsc.edu/ResTools/cmshp.html. Any sequence database that contains DNA or protein sequences corresponding to a gene or a segment thereof can be used for sequence analysis. Commonly employed databases include but are not limited to GenBank, EMBL, DDBJ, PDB, SWISS-PROT, EST, STS, GSS, and HTGS. Sequence similarity can be discerned by aligning the tag sequence against a DNA sequence database. Alternatively, the tag sequence can be translated into six reading frames; the predicted peptide sequences of all possible reading frames are then compared to individual sequences stored in a protein database such as the BLASTX program.

Parameters for determining the extent of homology set forth by one or more of the aforementioned alignment programs are well established in the art. They include, but are not limited to, p value, percent sequence identity and the percent sequence similarity. P value is the probability that the alignment is produced by chance. For a single alignment, the p value can be calculated according to Karlin et al. (1990) Proc. Natl. Acad. Sci. USA 87:2246. For multiple alignments, the p value can be calculated using a heuristic approach such as the one programmed in BLAST. Percent sequence identify is defined by the ratio of the number of nucleotide or amino acid matches between the query sequence and the known sequence when the two are optimally aligned. The percent sequence similarity is calculated in the same way as percent identity except one scores amino acids that are different but similar as positive when calculating the percent similarity. Thus, conservative changes that occur frequently without altering function, such as a change from one basic amino acid to another or a change from one hydrophobic amino acid to another are scored as if they were identical.

The polynucleotides can be conjugated to a detectable marker, e.g., an enzymatic label or a radioisotope for detection of nucleic acid and/or expression of the gene in a cell. A wide variety of appropriate detectable markers are known in the art, including fluorescent, radioactive, enzymatic or other ligands, such as avidin/biotin, which are capable of giving a detectable signal. In preferred embodiments, one will likely desire to employ a fluorescent label or an enzyme tag, such as urease, alkaline phosphatase or peroxidase, instead of radioactive or other environmental undesirable reagents. In the case of enzyme tags, colorimetric indicator substrates are known which can be employed to provide a means visible to the human eye or spectrophotometrically, to identify specific hybridization with complementary nucleic acid-containing samples.

The polynucleotides of the invention can comprise additional sequences, such as additional encoding sequences within the same transcription unit, controlling elements such as promoters, ribosome binding sites, and polyadenylation sites, additional transcription units under control of the same or a different promoter, sequences that permit cloning, expression, and transformation of a host cell, and any such construct as may be desirable to provide embodiments of this invention.

The polynucleotides embodied in this invention can be obtained using chemical synthesis, recombinant cloning methods, PCR, or any combination thereof. Methods of chemical polynucleotide synthesis are well known in the art and need not be described in detail herein. One of skill in the art can use the sequence data provided herein to obtain a desired polynucleotide by employing a DNA synthesizer or ordering from a commercial service.

Polynucleotides comprising a desired sequence can be inserted into a suitable vector, and the vector in turn can be introduced into a suitable host cell for replication and amplification. Polynucleotides can be introduced into host cells by any means known in the art. Cells are transformed by introducing an exogenous polynucleotide by direct uptake, endocytosis, transfection, f-mating or electroporation. Once introduced, the exogenous polynucleotide can be maintained within the cell as a non-integrated vector (such as a plasmid) or integrated into the host cell genome. Amplified DNA can be isolated from the host cell by standard methods. See, e.g., Sambrook, et al. (1989) *supra.* RNA can also be obtained from transformed host cell, or it can be obtained directly from the DNA by using a DNA-dependent RNA polymerase.

The present invention further encompasses a variety of gene delivery vehicles comprising the polynucleotide of the present invention. Gene delivery vehicles include both viral and non-viral vectors such as naked plasmid DNA or DNA/liposome complexes. Vectors are generally categorized into cloning and expression vectors. Cloning vectors are useful for obtaining replicate copies of the polynucleotides they contain, or as a means of storing the polynucleotides in a depository for future recovery. Expression vectors (and host cells containing these expression vectors) can be used to obtain polypeptides produced from the polynucleotides they contain. Suitable cloning and expression vectors include any known in the art, e.g., those for use in bacterial, mammalian, yeast and insect expression systems. The polypeptides produced in the various expression systems are also within the scope of the invention.

Cloning and expression vectors typically contain a selectable marker (for example, a gene encoding a protein necessary for the survival or growth of a host cell transformed with the vector), although such a marker gene can be carried on another polynucleotide sequence co-introduced into the host cell. Only those host cells into which a selectable gene has been introduced will grow under selective conditions. Typical selection genes either: (a) confer resistance to antibiotics or other toxins, e.g., ampicillin, neomycin, methotrexate; (b) complement autotrophic deficiencies; or (c) supply critical nutrients not available from complex media. The choice of the proper marker gene will depend on the host cell, and appropriate genes for different hosts are known in the art. Vectors also typically contain a replication system recognized by the host.

Suitable cloning vectors can be constructed according to standard techniques, or selected from a large number of cloning vectors available in the art. While the cloning vector selected may vary according to the host cell intended to be used, useful cloning vectors will generally have the ability to self-replicate, may possess a single target for a particular restriction endonuclease, or may carry marker genes. Suitable examples include plasmids and bacterial viruses, e.g., pBR322, pMB9, ColE1, pCR1, RP4, pUC18, mp18, mp19, phage DNAs, and shuttle vectors such as pSA3 and pAT28. These and other cloning vectors are available from commercial vendors such as Clontech, BioRad, Stratagene, and Invitrogen.

Expression vectors containing these nucleic acids are useful to obtain host vector systems to produce proteins and polypeptides. It is implied that these expression vectors must be replicable in the host organisms either as episomes or as an integral part of the chromosomal DNA. Suitable expression vectors include plasmids, viral vectors, including adenoviruses, adeno-associated viruses, retroviruses, cosmids, etc. A number of expression vectors suitable for expression in eukaryotic cells including yeast, avian, and mammalian cells are known in the art. One example of an expression vector is pcDNA3 (Invitrogen, San Diego, CA), in which transcription is driven by the cytomegalovirus (CMV) early promoter/enhancer. A particularly useful expression vector (system) is the baculovirus/insect system. Suitable vectors for expression in the baculovirus system include pBackPack9 (Clontech), pPbac and pMbac (Strategene). Adenoviral vectors are particularly useful for introducing genes into tissues *in vivo* because of their high levels of expression and efficient transformation of cells both *in vitro* and *in vivo.*

A vector of this invention can contain one or more polynucleotides encoding a polycystin transmembrane polypeptide. It can also contain polynucleotide sequences encoding other polypeptides that enhance, facilitate, or modulate the desired result, such as fusion components that facilitate protein purification, and sequences that increase immunogenicity of the resultant protein or polypeptide.

The vectors containing the polynucleotides of interest can be introduced into the host cell by any of a number of appropriate means, including electroporation, transfection employing calcium chloride, rubidium chloride, calcium phosphate, DEAE-dextran, or other substances; microprojectile bombardment; lipofection; and infection (where the vector is an infectious agent, such as vaccinia virus, which is discussed below). The choice of introducing vectors or polynucleotides will often depend on features of the host cell.

Once introduced into a suitable host cell, expression of a polycystin polypeptide can be determined using any assay known in the art. For example, presence of the polypeptide can be detected by RIA or ELISA of the culture supernatant (if the polypeptide is secreted) or cell lysates using antibodies reactive with the polycystin sequences or the fusion components (if also linked to the polypeptide).

Also embodied in the present invention are host cells transformed with polycystin polynucleotides as described above. Both prokaryotic and eukaryotic host cells may be used. Prokaryotic hosts include bacterial cells, for example E. *coli* and Mycobacteria. Among eukaryotic hosts are yeast, insect, avian, plant and mammalian cells. Host systems are known in the art and need not be described in detail herein. Examples of mammalian host cells include but not limited to COS, HeLa, and CHO cells. Baculovirus systems are preferred.

The host cells of this invention can be used, inter alia, as repositories of polycystin polynucleotides, or as vehicles for production of polycystin polynucleotides and polypeptides.

The polynucleotides and gene delivery vehicles of this invention have several uses. They are useful, for example, in expression systems for the production of polycystin or polycystin-related polypeptides. They are also useful as hybridization probes to assay for the presence of polycystin polynucleotide or related sequences in a sample using methods well known to those in the art. Further, the polynucleotides are also useful as primers to effect amplification of desired polynucleotides. The polynucleotides of this invention are also useful in pharmaceutical compositions including vaccines and for gene therapy.

### Uses of antibodies and polypeptides of the present invention

The antibodies and polypeptides embodied in this invention provide specific reagents that can be used in standard diagnostic procedures. Accordingly, the invention provides a method for detecting a polycystin-related polypeptide or tissue containing the polypeptide by contacting a sample suspected of containing the polypeptide with an antibody described herein. The presence of an antibody-antigen complex is indicative of the presence of the polycystin-related polypeptide.

Generally, to perform a diagnostic method of this invention, one of the compositions of this invention is provided as a reagent to detect a target in a sample with which it reacts. The target is supplied by obtaining a suitable biological sample from an individual for whom the diagnostic parameter is to be measured. Relevant biological samples are those obtained from individuals suspected of having polycystin kidney disease. A number of tissues are prone to develop cysts during the progression of PKD. These tissues include but are not limited to kidney, liver, spleen, brain, as well as gastrointestinal, cardiovascular and musculoskeletal tissues. Cells or tissue sample used for a diagnostic analysis encompass body fluid, solid tissue samples, tissue cultures or cells derived therefrom and the progeny thereof, and sections of smears prepared from any of these sources. Typically, cells are obtained by resection, biopsy or endoscopic sampling; the cells may be used directly, stored frozen, maintained or expanded in culture. Non-limiting examples of cell types useful for detecting the presence of polycystin and/or polycystin-related protein include epithelial cells, endothelial cells, neuronal cells, and interstitial fibroblasts. If desired, the target may be partially purified from the sample before the assay is conducted.

The reaction is performed by contacting the antibody with the sample under conditions that will allow a complex to form between the antibody and the target. The reaction may be performed in solution, or on a solid tissue sample, for example, using histology sections. The formation of the complex is detected by a number of techniques known in the art. For example, the antibodies may be supplied with a label and unreacted antibodies may be removed from the complex; the amount of remaining label thereby indicating the amount of complex formed.

The amount of the polypeptides that are immunologically reactive with the antibodies of the present invention can be quantified by standard quantitative immunoassays. If the protein is secreted or shed from the cell in any appreciable amount, it may be detectable in plasma or serum samples. Alternatively, the target protein may be solubilized or extracted from a solid tissue sample. Before quantification, the protein may optionally be affixed to a solid phase, such as by a blot technique or using a capture antibody. A number of immunoassay methods are established in the art for performing the quantitation. For example, the protein may be mixed with a predetermined non-limiting amount of the reagent antibody specific for the protein. The reagent antibody may contain a directly attached label, such as an enzyme or a radioisotope, or a second labeled reagent may be added, such as anti-immunoglobulin or protein A. For a solid-phase assay, unreacted reagents are removed by washing. For a liquid-phase assay, unreacted reagents are removed by some other separation technique, such as filtration or chromatography. The amount of label captured in the complex is positively related to the amount of target protein present in the test sample. Alternatively, a competitive assay in which the target protein is tested for its ability to compete with a labeled analog for binding sites on the specific antibody. In this case, the amount of label captured is negatively related to the amount of target protein present in a test sample. Results obtained using any such assay on a sample from a suspected polycyst-bearing source are compared with those from a non-polycystic source.

One important application of immunoassays employing the antibodies of the present invention is the determination of tissue and/or intracellular localization of the endogenous polycystin-related proteins. To discern the tissue distribution, frozen or fixed tissue sections and/or tissue homogenates can be stained using an above-described antibody at various concentrations. In testing each tissue for the expression of a polycystin-related proteins, it is also preferable to include a antibody known to react with a tissue-specific antigen that is differentially expressed in the tested tissue. Procedures for conducting immunohistological analysis are well established in the art and thus they are not detailed herein.

Also available in the art are a variety of techniques for examining the intracellular localization of a target polypeptide. Such techniques range from subcellular fractionation to cytoimmuno-staining and electron microscopy. Cell fractionation enables partial or complete separation of individual cellular organelles. An exemplary fractionation system is the hybrid Percoll/metrizamide discontinuous density gradient as described in (Storrie et al. (1990) Meth. Enzymol. 182:203-225). This gradient system allows the isolation of cell organelles including lysosomes, mitochondria and partial separation of plasma membrane from cytosol and organelles such as Golgi apparatus and endoplasmic reticulum. Cells suitable for such fractionation analysis include but are not limited to CHO cells and COS cells, that preferably overexpress the target polypeptides in order to enhance the detectable signal. After cell fractionation, various subcellular factions are typically assayed for the presence of the target polypeptide by immunoblotting with an appropriate antibody.

Cytoimmunostaining reveals the subcellular distribution of a target polypeptide by direct binding of an antibody specific for the target polypeptide present in a fixed cell. Typically, the cell to be stained is attached to a solid support to allow easy handling in the subsequent procedures. The second step for cell staining usually is to fix and permeabilize the cell to ensure free access of the antibody, although this step can be omitted when examining cell-surface antigens. After incubating cell preparations with the antibody, unbound antibody is removed by washing, and the bound antibody is detected either directly (if the primary antibody is labeled) or, more commonly, indirectly visualized using a labeled secondary antibody. In localizing a target polypeptide to a specific subcellular structure in a cell, co-staining with one or more marker antibodies specific for antigens differentially present in such structure is preferably performed. A battery of organelle specific antibodies is available in the art. Non-limiting examples include plasma membrane specific antibodies reactive with cell surface receptor HER2, ER specific antibodies directed to the ER resident protein Bip, and Golgi specific antibody α-adaptin. To detect and quantify the immunospecific binding, digital image analysis system coupled to conventional or confocal microscopy can be employed.

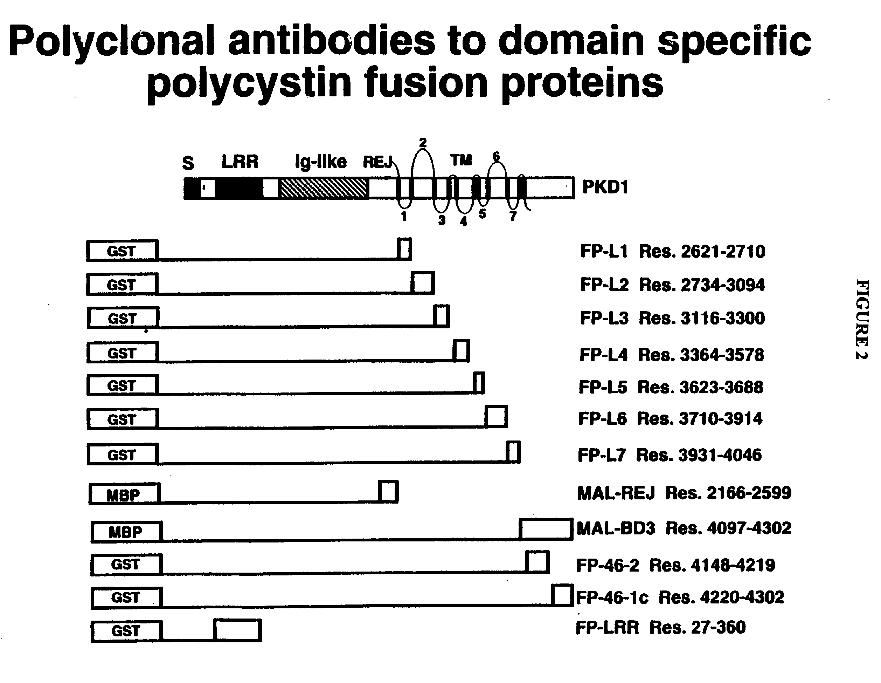

Applying the above described general techniques, a panel of approximately 8 domain-specific polyclonal antibodies as shown in Figures 1 and 2 detected in the crude membrane fractions of fetal kidney, liver as well as epithelial and astrocytoma cell lines, an endogenous polycystin-related protein of about 800 kD. The same antibodies recognized a smaller protein of approximately 600 kD in the membrane and cytosolic fractions of fetal brain. Expression of recombinant polycystin was characterized by immunoblotting and immunofluorescence analysis of COS cells, transiently expressing the full-length polycystin and four different truncated variants. Truncated polycystin was localized to the Golgi apparatus, while the full-length polycystin exhibited a different pattern of expression.

Discerning the tissue distribution and subcellular localization of polycystin-related proteins is of prime importance in elucidating the biological functions of these proteins. It can also be used for pathology studies. To determine whether the amount of a polycystin-related proteins, particularly the -600 kD or -800 kD proteins is representative of polycyst-bearing tissue or cell, a comparative immunoassay involving tissues or cells suspected to be affected by the disease are compared with a suitable control sample. The selection of an appropriate control cell or tissue is dependent on the sample cell or tissue initially selected and its phenotype which is under investigation. Whereas the sample cell is derived from a polycystic tissue, one or more counterparts of non-polycystic precursors of the sample cell can be used as control cells. Counterparts would include, for example, cell lines established from the same or related cells to those found in the sample cell population. Preferably, a control matches the tissue, and/or cell type the tested sample is derived from. It is also preferable to analyze the control and the tested sample in parallel.

### Kits comprising antibodies of the present invention

The present invention also encompasses kits containing the antibodies of this invention, preferably diagnostic kits. Kits embodied by this invention include those that allow someone to detect the presence or quantify the amount of a polycystin-related protein (particularly those having a molecular weight of ∼ 600 kD or about ∼800 kD) that are suspected to be present in a sample. The sample is optionally pre-treated for enrichment of the target being tested for. The user then applies a reagent contained in the kit in order to detect the changed level or alteration in the diagnostic component.

Each kit necessarily comprises the reagent which renders the procedure specific: a reagent antibody, used for detecting target protein; and optionally a reagent polypeptide, used as a control for the antibody, or used for detecting target antibody that may be present in a sample to be analyzed. Optionally, the antibody contained in the kits may be conjugated with a label to permit detection of any complex formed with the target in the sample. Alternatively, a second reagent is provided that is capable of combining with the first reagent after it has bound to its target and thereby supplying the detectable label. For example, labeled anti-rabbit IgG may be provided as a secondary reagent for use with the exemplified polyclonal antibodies. Labeled avidin may be provided as a secondary reagent when the primary reagent has been conjugated with biotin.

Each reagent can be supplied in a solid form or dissolved/suspended in a liquid buffer suitable for inventory storage, and later for exchange or addition into the reaction medium when the test is performed. Suitable packaging is provided. The kit can optionally provide additional components that are useful in the procedure. These optional components include, but are not limited to, buffers, capture reagents, developing reagents, labels, reacting surfaces, means for detection, control samples, instructions, and interpretive information. The kits can be employed to test a variety of biological samples, including body fluid, solid tissue samples, tissue cultures or cells derived therefrom and the progeny thereof, and sections or smears prepared from any of these sources. Diagnostic procedures using the antibodies of this invention can be performed by diagnostic laboratories, experimental laboratories, practitioners, or private individuals.

### Methods for modulating the biological activity of polycystin

Anti-fusion protein antibodies against three distant regions along the molecule were constructed. The production and characterization of antibodies against the N-terminal domain (anti-LRR) and C-terminal domain (anti-BD3) have previously been described (Ibraghimov-Beskrovnaya O. et al. (1997) Proc. Natl. Acad. Sci. USA 94:6397-6402). Anti-L2 antibody, which is positioned in the middle region of polycystin-1 in the REJ domain was constructed as described above.

The specificity of the anti-polycystin-1 antibodies was examined using recombinant polycystin-1.

Anti-L2 antibody specificity against the GST-L2 fusion protein expressed in bacterial cells was tested. Anti-L2 antibody specifically recognized the L2 domain when fused to GST. Additionally, these antibodies were able to precipitate *in vitro* translated polycystin-1 specifically. Thus, the antibodies used in this study were rigorously characterized for their ability to immunoprecipitate *in vitro* translated polycystin-1 as well as by Western and immunofluorescence analysis of recombinant polycystin-1.

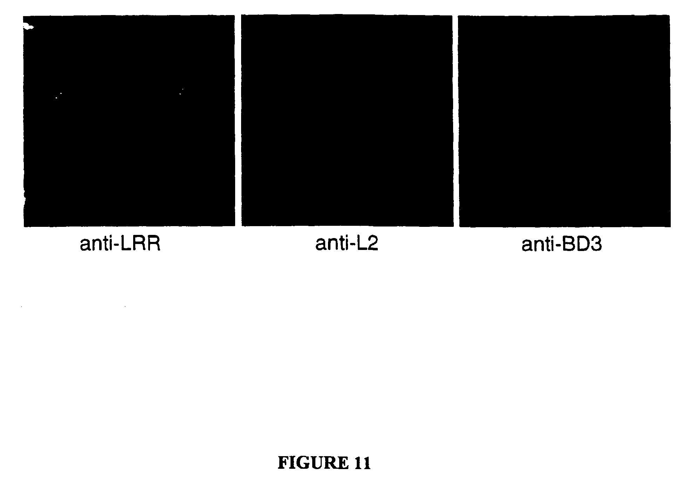

To determine the subcellular localization of endogenous polycystin-1 in epithelial cells, immunostaining of polycystin-1 in MDCK cells was performed with antibodies. The antibodies used were to the N-terminal region (anti-LRR), C-terminal region (anti-BD3) and to the REJ domain in the middle portion of the protein (anti-L2). All antibodies showed clearly recognizable membrane staining at sites of cell-cell contact (Figure 11). No staining was observed with the secondary antibody alone as control. Isolated cells and free cell borders of contacting cells did not localize polycystin-1 at the membrane, although some intracellular staining can be seen. These data suggest that the compartmentalization of polycystin-1 is dynamic and that trafficking of polycystin-1 between the cytoplasm and plasma membrane compartments is a function of cell contact.

The polycystic kidney disease 1 (PKD1) gene encodes a novel protein with multiple cell recognition domains. Hughes J. et al. (1995) Cell 10:151-159. The analysis of the three-dimensional structure of a single repeat showed that it is not a true member of Ig superfamily, although it has a characteristic ß-sandwich topology. Bycroft M. et al. (1999) EMBO J. 18:297-305. Domains with this Ig-like fold are present in proteins as diverse as matrix proteins, receptors and enzymes, and in each case they have been shown to interact with extremely different ligands varying from small peptides (e.g., HLA) to giant proteins (e.g., titin oligomer). Bork A. et al. (1994) J. Mol. Biol. 242:309-320.

Using antibodies against three different regions of polycystin-1: N-terminal (LRR), C-terminal, and the middle region (REJ), the experiments described herein clearly showed that polycystin-1 was predominantly expressed at sites of cell-cell contact in kidney epithelial cells, as was the case for endothelial cells. The homophilic binding potential of several Ig-like domains, i.e., Ig^{a}, Ig^{b} and Ig^{c} containing 4, 5 and 6 domains, as clusters were analyzed as described below. Each region was translated *in vitro* and tested for the ability to bind to each region including itself in the form of immobilized fusion protein. The binding properties of all combinations were quantitatively analyzed as a percentage of binding of *in vitro* translated protein. In this type of assay the fusion proteins are present in a vast excess compared to the amount of the translated probe. Therefore, theoretically almost all of the translated probe should bind to immobilized fusion protein, even if binding is weak. Phizicky, E.M. & Fields, S. (1995) Microbiological Reviews 59:94-123. In practice, deviations from quantitative binding occur if not all of the immobilized protein or/and *in vitro* translated probe is functionally active. Nevertheless, a functionally relevant interaction should result in significant retention of ligand. For example, estimates from affinity chromatography binding experiments on the N-NusA, NusA-RNA polymerase and RAP30/74-RNA polymerase II interactions indicate that at least 50% of these proteins are available for binding. Formoza, T. et al. (1991) Meth. Enzymol. 208:24-45.

Strong homophilic interactions were detected between the Ig-like domains, which are calcium independent. The strongest interaction was detected for the combination Ig^{c}-Ig^{c}, where the bound fraction constituted up to 90%. The least efficient interaction, characterized by 20% binding was detected for the Ig^{a}-Ig^{a} and Ig^{a}-Ig^{b} combinations. Ig^{b}-Ig^{b}, Ig^{b}-Ig^{c} combinations demonstrated intermediate binding ranging from 25-45%. The observed difference in binding capacities could be due to the different number of Ig-domains in each construct, so that the higher number of repeats results in stronger binding because of higher avidity. It could also be due to the cooperative nature of this interaction. The homophilic binding of polycystin-1 resembles that of chick NCAM where all of the five Ig-like domains are involved in homophilic interactions. Ranheim T.S. et al. (1996) Proc. Natl. Acad. Sci. USA 93:4071-4075. It is possible that the homophilic interactions described in this study might mediate homodimerization in addition to homophilic adhesion at intercellular contacts. A similar mechanism was shown to be important in the functioning of the PECAM-1 protein and modulating its ligand binding state (homophilic or heterophilic). Sun J. et al. (1996) J. Biol. Chem. 271:18561-18570. In addition, homotypic binding between the extracellular domains of cadherins mediates formation of complexes between parallel-oriented molecules on single cells and between cells, which is thought to cooperatively enhance adhesion. Brieher W.M. et al. (1996) J. Cell Biol. 135:487-496. Similarly, the data shown herein suggest that cis interactions between polycystin-1 molecules, mediating homodimerization on the same membrane might coexist with trans-interactions between opposing molecules at the site of cell-cell contact.

To adequately assess the significance of the Ig-like domain homophilic interactions under consideration, they were compared them side by side with known interactions. One of those was the interaction between p53 and SV40 large T-antigen, which is known to be functionally significant. Lane D.P. et al. (1979) Nature 278:261-262 and Iwabuchi K. et al. (1993) Oncogene 8:1693-1696. The bound fraction of T-antigen comprised approximately 45% of the total probe in this system. The interaction between the PKD1 and PKD2 gene products also was used as a reference. Quian F. et al. (1997) Nature Genetics 16:179-183 and Tsiokas L. et al. (1997) Proc. Natl. Acad. Sci. USA 94:6965-6970. This interaction was initially identified by the two-hybrid assay and was further characterized using the *in vitro* binding assay. Approximately 1.5% of the input polycystin-1 probe bound to immobilized polycystin-2, while 6% of the labeled ligand was bound in the reverse combination. Quian F. et al. (1997) Nature Genetics 16:179-183. Similarly, a weak PKD2-PKD1 gene product interaction was detected which never exceeded ∼1% of binding in different buffer compositions. Thus, the strength of the homophilic interactions between the various Ig-like regions of polycystin-1 as *measured in vitro* is more comparable to the known functionally significant p53-T antigen binding rather than to the weaker and likely transient interaction between polycystin-1 and -2.

The importance of this biochemical binding assay results was tested *in vivo* by assessing the effect of soluble Ig-like domains on cell adhesion using both cell monolayers and cells in suspension. It was shown that soluble Ig-like domains perturb *in vivo* intercellular adhesion in MDCK cell monolayers, suggesting that they are directly involved in intercellular adhesion. It was likewise shown that soluble Ig-like domains can interfere with cellular adhesion using a cell aggregation assay.

The formation and progression of ADPKD cysts is characterized by increased cell proliferation, resulting in expansion of the epithelium, which displays a relatively undifferentiated appearance. Grantham J. (1996) Amer. J. Kidney Diseases 28:788-803 and Avner E.D. (1993) J. Cell Sci. 17:217-222. The role of polycystin-1 in mediating cell-cell interactions, where such interactions are fundamental for cellular functions of proliferation, differentiation and maturation, is supported by a recent study of a targeted PKD1 mutation in mice. Lu W. et al. (1997) Nature Genetics 17:179-181. This study demonstrates that polycystin-1 is critical in the establishment and maturation of normal tubular architecture. Lu W. et al. (1997), *supra.* It has been shown that the expression of polycystin-1 is continued into adult life at a lower level, where its functional activity might be required for cells to remain tightly associated in the epithelium. Peters D.J.M. et al. (1996) Laboratory Investigation 75:221-230; Ibraghimov-Beskrovnaya O. et al. (1997) Proc. Natl. Acad. Sci. USA 94:6397-640; Weston B.S. et al. (1997) Histochemical Journal 29:847-856 (1997); and Ward C.J. et al. (1996) Proc. Natl. Acad. Sci. USA 93:1524-1528. In addition, it is known that cell adhesion proteins play an important role in intercellular signaling. Gumbiner B.M. (1996) Cell 84:345-357. The results presented herein show that the loss of intercellular interactions due to a mutated polycystin-1 can be an important step in molecular cystogenesis.

Thus, in view of the above, this invention provides a method for modulating cell-cell adhesion in a suitable tissue, comprising delivering to the tissue an effective amount of an agent that modulates the binding of polycystin in the tissue. In one aspect, the modulation of cell-cell or cell-matrix adhesion is a reduction of cell-cell or cell-matrix adhesion. In another aspect, the modulation of cell-cell or cell-matrix adhesion is an increase or to enhance cell-cell or cell-matrix adhesion mediated by polycystin in a suitable tissue. As used herein, a "suitable tissue" includes any tissue which polycystin, i.e., polycystin-1 or polycystin-2, is expressed as been described above.

In one aspect, the agent is any agent that inhibits polycystin-1 mediated cell-cell or cell-matrix adhesion. Such agents include, but are not limited to, agents such as the antibodies described herein that bind to the Ig-like domains of polycystin, polycystin fragments comprising the Ig-like domains and agents that inhibit the expression of polycystin, e.g., polycystin-1 or polycystin-2, in a cell. Such agents include, but are not limited to antisense polycystin DNA and ribozymes that specifically recognize or cleave polycystin RNA in a cell.

One of skill in the art is enabled to make and use the agents noted above using the methods and compositions described herein alone or in combination with the methods known to those of skill in the art.

Alternatively, this invention also provides methods to promote cell-cell or cell-matrix adhesion in a tissue by delivering to the cell or tissue an effective amount of polycystin-1 to the cell or a polypeptide comprising an Ig-like domain of polycystin to the cell or tissue. The polycystin is delivered in the form of a polynucleotide or polypeptide or protein. In addition, one can restore normal cell-cell or cell-matrix adhesion is a tissue containing soluble, mutated polycystin by removing or binding the mutated polycystin using the anti-polycystin antibodies described herein as well as those known in the art.

The methods of this invention can be practiced *in vitro, in vivo* or *ex vivo.* When practiced *in vitro,* the methods provides screens for therapeutic agents that augment or inhibit the biological activity of wild-type or mutated polycystin in a cell or tissue. To practice the screen, suitable cell cultures or tissue cultures are first provided. The cell can be a cultured cell or a genetically modified cell in which wild-type or mutated polycystin transmembrane regions are expressed on the cell surface. Alternatively, the cells can be from a tissue biopsy. The cells are cultured under conditions (temperature, growth or culture medium and gas (CO₂)) and for an appropriate amount of time to attain exponential proliferation without density dependent constraints. It also is desirable to maintain an additional separate cell culture; one which does not receive the agent being tested as a control.

As is apparent to one of skill in the art, suitable cells may be cultured in microtiter plates and several agents may be assayed at the same time by noting genotypic changes or phenotypic changes.

When the agent is a composition other than a DNA or RNA nucleic acid molecule, the suitable conditions may be by directly added to the cell culture or added to culture medium for addition. As is apparent to those skilled in the art, an "effective" amount must be added which can be empirically determined.

For the purposes of this invention, an "agent" is intended to include, but not be limited to a biological or chemical compound such as a simple or complex organic or inorganic molecule, a peptide, a protein (e.g. antibody) or an oligonucleotide (e.g. anti-sense). A vast array of compounds can be synthesized, for example oligomers, such as oligopeptides and oligonucleotides, and synthetic organic compounds based on various core structures, and these are also included in the term "agent". In addition, various natural sources can provide compounds for screening, such as plant or animal extracts, and the like. It should be understood, although not always explicitly stated that the agent is used alone or in combination with another agent, having the same or different biological activity as the agents identified by the inventive screen. The agents and methods also are intended to be combined with other therapies.

When the agent is a nucleic acid, it can be added to the cell cultures by methods well known in the art, which includes, but is not limited to calcium phosphate precipitation, microinjection or electroporation. Alternatively or additionally, the nucleic acid can be incorporated into an expression or insertion vector for incorporation into the cells. Vectors that contain both a promoter and a cloning site into which a polynucleotide can be operatively linked are well known in the art. Such vectors are capable of transcribing RNA *in vitro* or *in vivo,* and are commercially available from sources such as Stratagene (La Jolla, CA) and Promega Biotech (Madison, WI). In order to optimize expression and/or in *vitro* transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression. Examples of vectors are viruses, such as baculovirus and retrovirus, bacteriophage, adenovirus, adeno-associated virus, cosmid, plasmid, fungal vectors and other recombination vehicles typically used in the art which have been described for expression in a variety of eukaryotic and prokaryotic hosts, and may be used for gene therapy as well as for simple protein expression.

One can determine if the object of the method, i.e., modulation of cell-cell or cell-matrix adhesion has been achieved by noting phenotypic change in the cell as described below or by alteration of transcript expression. Kits containing the agents and instructions necessary to perform the screen and *in vitro* method as described herein also are claimed.

When the subject is an animal such as a rat or mouse, the method provides a convenient animal model system which can be used prior to clinical testing of the therapeutic agent. It also can be useful to have a separate negative control group of cells or animals which are healthy and not treated, which provides a basis for comparison.

These agents of this invention and the above noted compounds and their derivatives may be used for the preparation of medicaments for use in the methods described herein.

In a preferred embodiment, an agent of the invention is administered to treat a pathology associated with abnormal polycystin expression such as PKD. Various delivery systems are known and can be used to administer a therapeutic agent of the invention, e.g., encapsulation in liposomes, microparticles, microcapsules, expression by recombinant cells, receptor-mediated endocytosis (see, e.g., Wu and Wu (1987) J. Biol. Chem. 262:4429-4432), construction of a therapeutic nucleic acid as part of a retroviral or other vector, etc. Methods of delivery include but are not limited to intra-arterial, intra-muscular, intravenous, intranasal, and oral routes. In a specific embodiment, it may be desirable to administer the pharmaceutical compositions of the invention locally to the area in need of treatment; this may be achieved by, for example, and not by way of limitation, local infusion during surgery, by injection, or by means of a catheter.

The agents identified herein as effective for their intended purpose can be administered to subjects or individuals susceptible to or at risk of developing a disease associated with abnormal polycystin expression such as PKD. When the agent is administered to a subject such as a mouse, a rat or a human patient, the agent can be added to a pharmaceutically acceptable carrier and systemically or topically administered to the subject. Therapeutic amounts can be empirically determined and will vary with the pathology being treated, the subject being treated and the efficacy and toxicity of the agent.

Administration *in vivo* can be effected in one dose, continuously or intermittently throughout the course of treatment. Methods of determining the most effective means and dosage of administration are well known to those of skill in the art and will vary with the composition used for therapy, the purpose of the therapy, the target cell being treated, and the subject being treated. Single or multiple administrations can be carried out with the dose level and pattern being selected by the treating physician. Suitable dosage formulations and methods of administering the agents can be found below.

An agent of the present invention also referred to herein as the active ingredient, may be administered for therapy by any suitable route including oral, rectal, nasal, topical (including transdermal, aerosol, buccal and sublingual), vaginal, parental (including subcutaneous, intramuscular, intravenous and intradermal) and pulmonary. It will also be appreciated that the preferred route will vary with the condition and age of the recipient, and the disease being treated.

Ideally, the agent should be administered to achieve peak concentrations of the active compound at sites of disease. This may be achieved, for example, by the intravenous injection of the agent, optionally in saline, or orally administered, for example, as a tablet, capsule or syrup containing the active ingredient. Desirable blood levels of the agent may be maintained by a continuous infusion to provide a therapeutic amount of the active ingredient within disease tissue. The use of operative combinations is contemplated to provide therapeutic combinations requiring a lower total dosage of each component antiviral agent than may be required when each individual therapeutic compound or drug is used alone, thereby reducing adverse effects.

It should be understood that in addition to the ingredients particularly mentioned above, the formulations of this invention may include other agents conventional in the art having regard to the type of formulation in question, for example, those suitable for oral administration may include such further agents as sweeteners, thickeners and flavoring agents. It also is intended that the agents, compositions and methods of this invention be combined with other suitable compositions and therapies.

The following examples are intended to illustrate, but not limit this invention.

### EXAMPLES

### EXPERIMENT NO 1 - PRODUCTION OF ANTI-POLYCYSTIN ANTIBODIES

### Example 1: Production and characterization of polyclonal antibodies raised against the transmembrane domain of polycystin

A panel of seven GST-fusion proteins containing sequences corresponding to a specific loop region (see Figure 2) and one MBP-fusion protein comprising sequences outside the loop region of the polycystin transmembrane domain were expressed in E. *coli* and used to immunize rabbits. The production and characterization of the anti-loop 4 antibodies were detailed below.

A fragment ofpolycystin cDNA corresponding to amino acids 3364-3578 was cloned into pGEX vector (Pharmacia) for production of FP-L4 fusion protein *E. coli* (Figure 2). *E. coli* DH5 alpha cells carrying this construct were grown overnight, diluted 1:10 and induced with 0.1 mM IPTG for 3 hours. Fusion protein was isolated as suggested by the manufacturer (Pharmacia) and injected into two rabbits for production of polyclonal antisera. Antibodies were shown to specifically recognize corresponding immunogen (FP-L4) on western blot. In addition, produced anti-FP-L4 antibodies specifically recognized truncated polycystin, expressed in baculovirus/insect system.

### Example 2: Fractionation of tissue homogenates

To separate the particulate fractions (or crude membranes) from the cytosolic fractions, tissues were homogenized in 7 volume of homogenization buffer containing 10 mM HEPES, pH 7.4, 0.25 M sucrose, 0.5 mM MgCl₂, 0.1 mM PMSF, 0.75 mM benzamidine, 1 µg/ml aprotinin, 1 µg/ml leupeptin, and 1 µg/ml pepstatin. The homogenates were then centrifuged at 1,100 x g for 15 min at 4° C, and the supernatant was filtered through cheesecloth. Total tissue membranes were pelleted by centrifugation at 140,000 x g for 1 hour at 4° C and the supernatants were collected as the cytosolic fractions.

The fractionation of subcellular structures was carried out by differential centrifugation. Homogenates prepared as described above were first centrifuged at 600 x g for 10 min at 4°C. The resulting supernatant S600_{I} was collected, and the pellet P600_{I} was resuspended in homogenization buffer and then centrifuged under the same condition to yield the supernatant S600_{II} and the pellet P600_{II} fractions. Fraction S600_{I} containing the cytosolic contents as well as fraction S600_{II} containing the membrane structures of the cells were then combined and subjected to high speed centrifugation at 150,000 x g for 10 min at 4°C. The resulting pellet, P15K, containing large organelles including mitochondria and lysosomes were collected, and the supernatant S15K was further fractionated at 150,000 x g for 60 min at 4° C to yield fraction S150K and P150K. Whereas S150K contains cytosolic components, P150K contains low density membrane structures such as plasma membrane, Endoplasmic reticulum and Golgi apparatus. The presence of a polycystin-related protein in various cell fractions was then determined by immunoassays employing one or more of the antibodies described herein. A polycystin-related protein having a molecular weight higher then 200 kD was predominantly detected in the membrane fractions P15K and P150K and not in the cytosolic fraction S150K of both the kidney and liver homogenates. This suggests that the polycystin-related protein expressed in these two tissues is associated with one or more cellular membrane structures, including plasma membrane, mitochondria, lysosomes, Endoplasmic reticulum and Golgi apparatus. Fractionation of fetal brain tissues, however, revealed that a polycystin-related protein having a lower molecular weight than the one expressed in the kidney and liver was associated with both the cytosolic fraction (S150K) and the microsomal fraction (P150K).

To further investigate the possibility that the polycystin-related protein expressed in the kidney is an integral membrane protein, membrane fractions was subjected to a "high salt" wash using, e.g., 0.3 M potassium chloride. The membrane bound polycystin-related protein was resistant to "high salt" washing. No polycystin-related protein expressed in the kidneys was dislodged from the membrane and released to the supernatant fraction (S150K KCl) after high speed centrifugation. This result suggests that the polycystin-related protein expressed in the kidneys is tightly bound to the cellular membranes, and likely to be an integral membrane protein.

### Example 3: Gel electrophoresis and immunoblotting

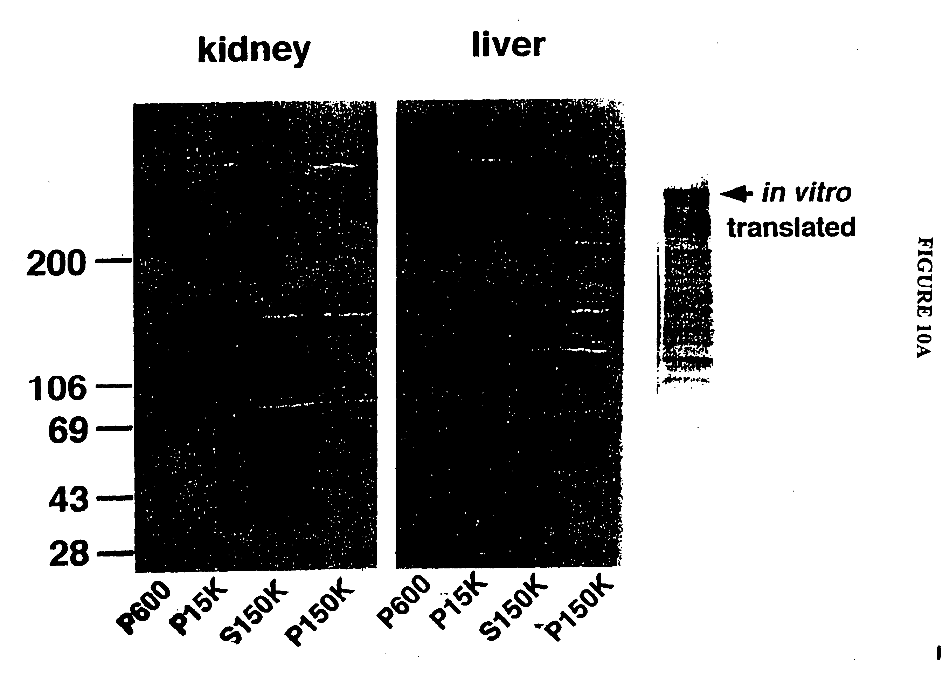

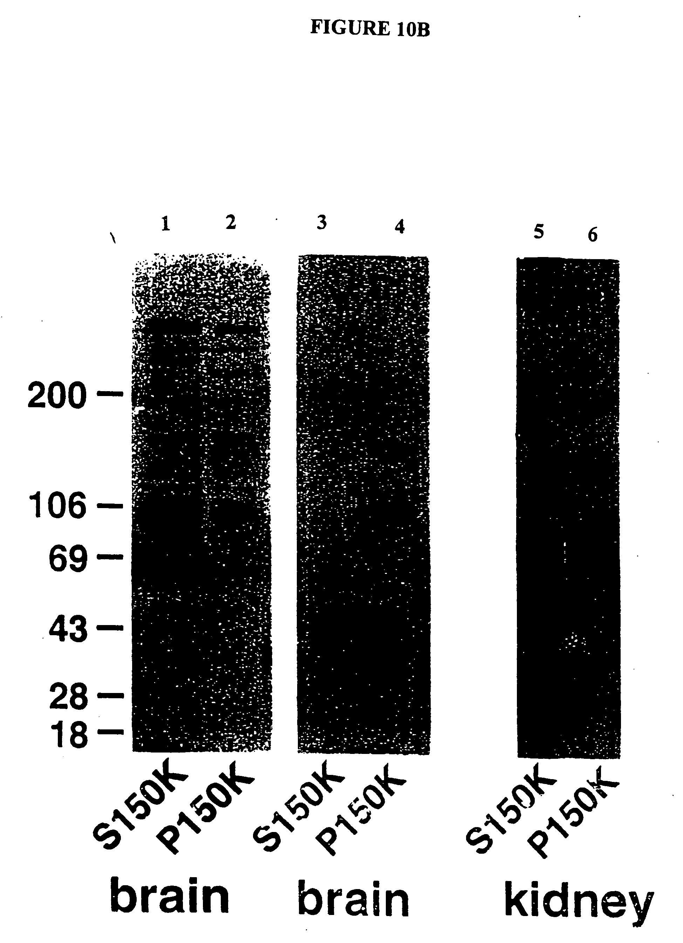

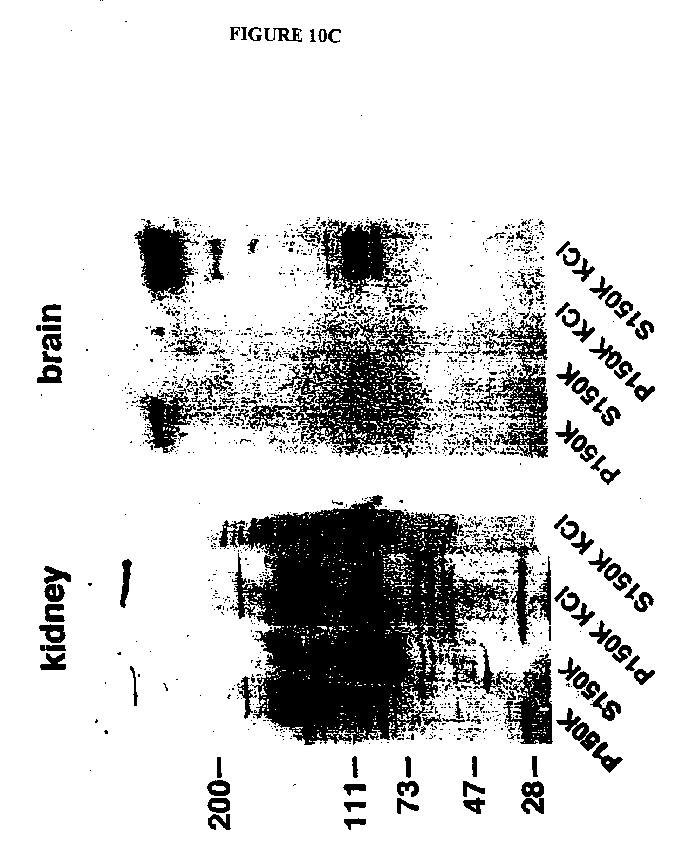

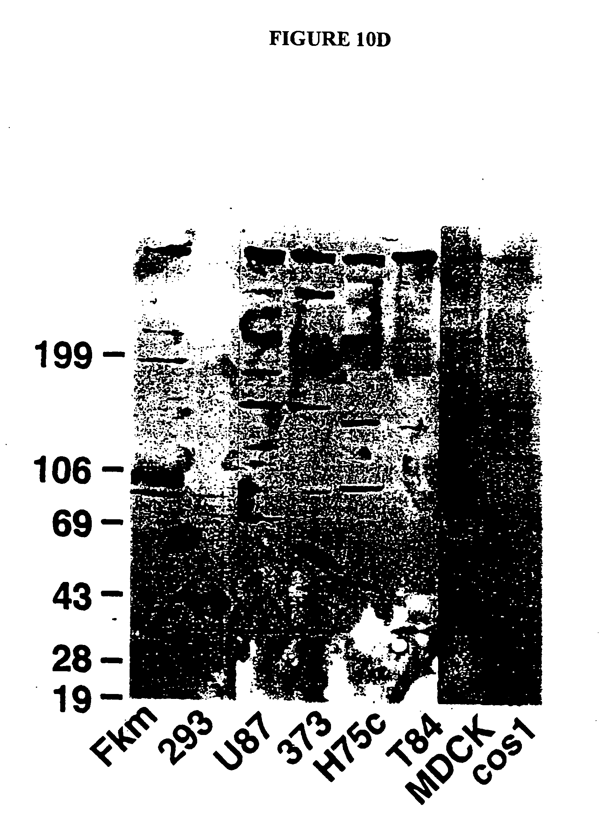

Proteins of each tissue fraction were separated on 3-12% gradient SDS polyacrylamide gels. Transfer of proteins to nitrocellulose was performed by electroblotting. For immunoblotting membranes were pre-blocked in Blotto (5% nonfat dry milk in PBS, pH 7.4) for 1 hour, then incubated overnight with 1:1 00 diluted anti-FP-L4 antibodies. After washing membranes three times for 10 min in Blotto, immunoblots were incubated with 1:1000 diluted peroxidase-conjugated goat anti-rabbit IgG for 1 hour, washed and developed by ECL. A protein band of ∼ 800 kD was detected in the membrane fractions of kidney and liver tissues. Similar - 800 kD band was also detected in a number of cell lines (see Figure 10D). Another protein band of∼600 kD was detected in the membrane and cytosolic fractions of the fetal brain homogenates.

### Example 4: Polycystin expression in baculovirus/insect system and in COS cells.

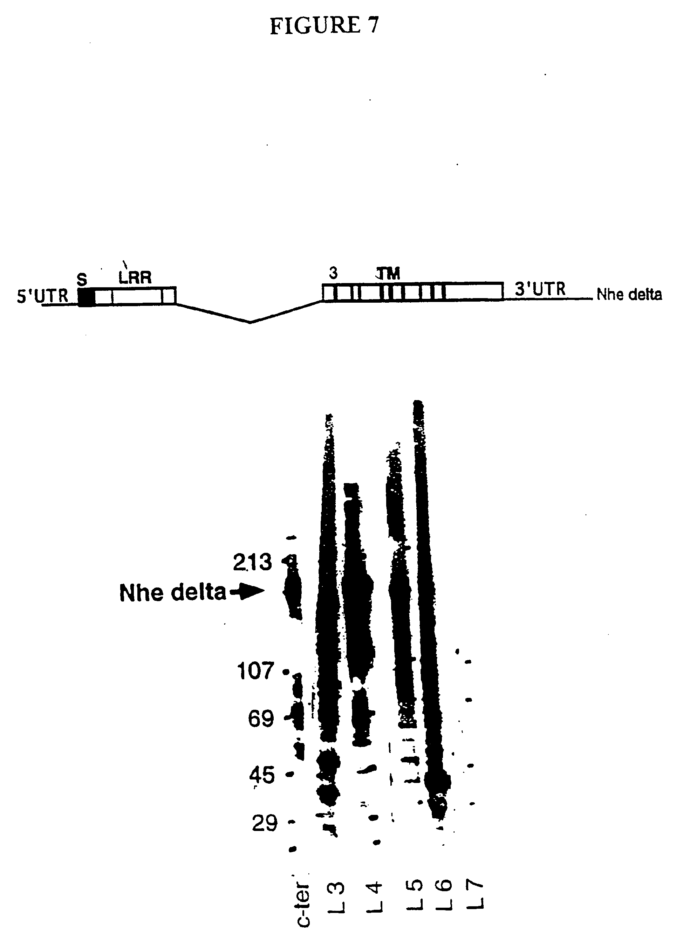

Nhe-delta mutant deleted with amino acids 290-2960 (Figure 3) was generated for expression in baculovirus/insect system. Polycystin cDNA was cloned into pBacPAK9 transfer vector (Clontech). Insect cells Sf21 were cotransfected with transfer-polycystin plasmid and viral DNA and incubated for 72 hr. Several individual recombinant virus plaques were analyzed for recombinant protein production. Total cell lysates infected with individual plaques were separated by SDS-PAGE and analyzed by immunoblotting with anti-Loop4 antibodies. Expected immunoreactive band of∼170 kD, corresponding to the truncated polycystin was detected (see Figure 7).

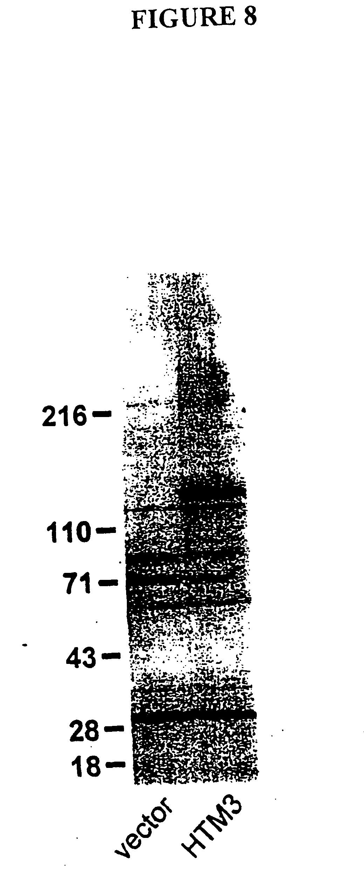

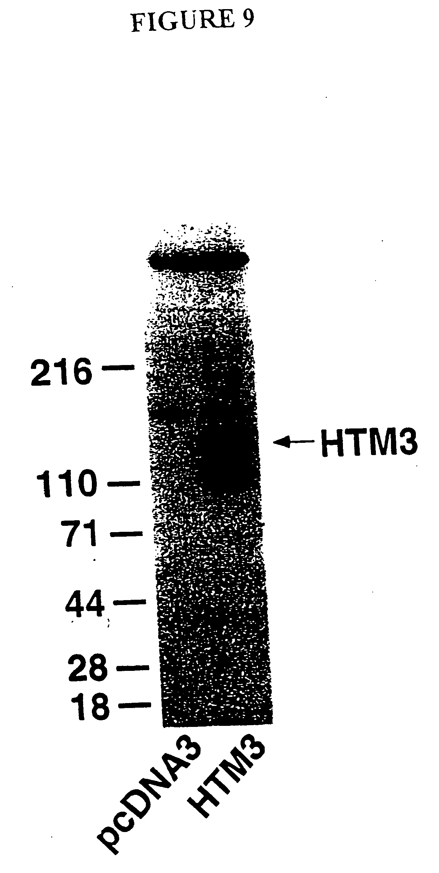

Another deletion mutant (HTM3) containing the C-terminal portion of polycystin that encompasses most of the transmembrane domain and the entire intracellular domain was cloned into an expression vector. Transient expression of the truncated polycystin was detected by immunoblotting cell lysates obtained from the COS 1 cells transfected with the vector (Figures 8-9). No expression of the recombinant protein was found in the COS 1 cells transfected with a control vector.

### EXPERIMENT NO 2: CELL-CELL/CELL-MATRIX ADHESION

### Example 5: Anti-polycystin-1 antibodies preparation

All antibodies were raised in rabbits against fusion proteins representing different domains of polycystin-1. Anti-LRR (Res. 27-360) and anti-BD3 (Res. 4097-4302) were affinity purified as described. Anti-L2 antibody was produced against GST fusion protein containing part of REJ domain of polycystin-1 (Res. 2714-3074).

### Example 6: Expression of recombinant polycystin-1 in baculovirus/insect cell systems

Truncated polycystin-1 was expressed by using BacPAK TM Baculovirus Expression System (Clontech) according to the manufacturer's instructions. Briefly, PKD1 cDNA inserts HTM3 and Nhe delta were subcloned into pBacPAK9 transfer vector and co-transfected with BacPAK6 viral DNA into Sf21 insect cells. Individual plaques from the supernatant co-transfection medium were analyzed and selected for the high level of polycystin-1 protein production as assayed by Western blotting.

### Example 7: Immunofluoresence

MDCK cells (source) or baculovirus infected Sf21 cells were grown on glass coverslips and immunostained as described in Ibraghimov-Beskrovnaya, O. et al. (1997) Proc. Natl. Acad. Sci. 94:6397-6402. The primary antibodies were used at a dilution of 1:100 followed by incubation with FITC labeled goat anti-rabbit secondary antibody at a dilution 1:200. Cells were examined using a Zeiss Axioplan microscope.

### Example 8: Production of fusion proteins for in vitro binding assay

The cluster of Ig-like domains of polycystin-1 was subdivided into three constructs: Ig^{a} (domains II-V (amino acids 843-1200)), Ig^{b} (domains VI-X (amino acids 1205-1625)) and Ig^{c} (domains XI-XVI (amino acids 1626-2136)) and subcloned into pGEX-1 vector (Pharmacia) for production of GST fusion proteins designated GST-Ig^{a}, GST-Ig^{b} and GST-Ig^{c}, respectively. The cDNA fragments for each construct were synthesized by PCR using as template the full-length human PKD1 cDNA described previously in Ibraghimov-Beskrovnaya O. et al. (1997) Proc. Natl. Acad. Sci. 94:6397-6402. The C-terminal region of polycystin-1 (MBP-PKD1) (Res. 4077-4302) was constructed as an MBP fusion protein by cloning in the expression vector pMALc2 (NEB). The GST-p53 construct (Res.73-390) was produced as GST-fusion protein. The GST fusion proteins were purified from supernatants by affinity chromatography on Glutathione-Sepharose (Pharmacia) as recommended by the manufacturer.

### Experiment 9: In vitro translation probes

Translation of the PKD1 constructs *in vitro* was performed using the TNT Coupled Reticulocyte Lysate System (Promega) as recommended by the manufacturer. The Ig-like domains of polycystin-1: Ig^{a} (domains II-V), Ig^{b} (domains VI-X) and Ig^{c} (domains XI-XVI) were subcloned downstream of the oligo GTAATACGACTCACTATAGGGCGAGCCACCATGG (SEQ ID NO:3), containing the T7 RNA polymerase promoter (bold) followed by an AUG initiation codon in a Kozak consensus context (underlined). This oligo was inserted between the BamHI and EcoRI sites of the pGEX-4T-1 vector (Pharmacia) downstream of GST coding region, such that the same construct can be used for either GST fusion protein production or for the *in vitro* translation of the insert without the GST portion. ³⁵S-PKD2 probe (Res. 657-968) and ³⁵S-T-antigen probe (res. 87-708) were generated in the same manner.

GST-fusion proteins or GST alone were immobilized individually onto Glutathione Sepharose (Pharmacia). MBP-PKD1 fusion protein or MBP-lacZ as control were immobilized onto amylose resin (NEB). Twenty (20) µL of beads witch∼10 µg of immobilized fusion proteins were used for each binding reaction. Approximately 10 µl of *in vitro* translated ³⁵S-labeled probe were incubated for 3hours at room temperature with immobilized fusion proteins in 0.1 ml of binding buffer (10 mM HEPES, pH 7.4,100 mM NaCl, 1 mM CaCI₂, 2 mM MgCl₂, 0.75 mM benzamidine, 0.1 mM PMSF) and washed with 20 column volumes of the same buffer. The polycystin-2 and polycystin-1 interaction assay was also performed in another buffer (10 mM Tris, pH 7.4, 200 mM NaCl,1 mM EDTA). The ³⁵S-translated material bound to the beads was resolved by SDS-PAGE with input ³⁵S probe run in parallel. The gels were exposed to film (X-Omat AR, Kodak) as well as quantified using a PhosphorImager with ImageQuant (v. 3.2) software (Molecular Dynamics). Only bands representing the full-length product *of in vitro* translation were used for quantification in each binding reaction and bound fractions were estimated as percentage of input of ³⁵S translated probe.

SDS-PAGE was carried out on 3-12% or 5-15% gradient gels in the presence of 1% 2-mercaptoethanol and transferred to nitrocellulose for immunoblot analysis as described 43 Primary anti-polycystin-1 antibodies were used at a dilution 1:100 and secondary goat anti-rabbit-HRP antibodies (Boehringer Mannheim) were used at a dilution 1:1000.

### Experiment 10: Disruption of cell-cell adhesion in cell monolayers and aggregation assay

The disruption of intercellular adhesion was performed by the method of Wheelock et al. (1987) J. Cell Biochem. 34:187-202. MDCK cells were grown 24 hours to 70% confluency in media with 10% fetal bovine serum. The complete media was replaced with control serum-free media alone or with media containing either GST carrier protein or GST-Ig^{a}, GST-Ig^{b} and GST-Ig^{c} fusion proteins (1 nM each) as described above. Cells were incubated for 30 hours and live cell images were collected using a Nikon Eclipse 200 microscope equipped with a Sony CCD/RGB camera DXC-151 and Scionimage 1.62a software (Scion Corporation).

The aggregation assay was performed as described in DeLisser et al. (1993) J. Biol. Chem. 268:16037-16046. with minor modifications. Briefly, MDCK cells were plated at 5x10⁶ cells/10cm plate and grown for 24 hours. Cells were harvested by incubation in PBS with 10 mM EDTA for 15 min followed by incubation with 0.01% trypsin for 2 min. After washing the cells were resuspended at ∼1x10⁶/ml in serum free media alone or media with GST protein or with GST-Ig^{a}, GST-Ig^{b} and GST-Ig^{c} at a concentration of 7 nM each. Cells were transferred to a 24-well plastic tray, previously blocked with 3% BSA in PBS and rotated at 100 rpm at 37°C for 1.5 hour and images of live cells were collected as described above.

## Claims

1. An isolated antibody raised against an epitope, the epitope having the amino acid sequence consisting of amino acid residues 843 to 1200, amino acid residues 1205 to 1625, or amino acid residues 1626 to 2136, of SEQ ID NO: 2.

2. An isolated antibody of claim 1, wherein the antibody is selected from a polyclonal antibody, monoclonal antibody or a polyclonal or monoclonal antibody labeled with a detectable label.

3. A composition comprising a carrier and an antibody of any one of claims 1 to 2.

4. A hybridoma cell line that produces a monoclonal antibody as claimed in claim 2.

5. An isolated antibody of any one of claims 1 to 2, wherein the epitope is expressed in a tissue selected from the group consisting of kidney, brain, liver, and neuronal tissues.

6. A recombinant polypeptide having the amino acid sequence consisting of amino acid residues 843 to 1200, amino acid residues 1205 to 1625, or amino acid residues 1626 to 2136, of SEQ ID NO: 2.

7. A composition comprising a carrier and a polypeptide of claim 6.

8. An isolated polynucleotide encoding the recombinant polypeptide of claim 6.

9. A gene delivery vehicle comprising the polynucleotide of claim 8.

10. A host cell transformed with the isolated polynucleotide of claim 8.

11. A method of detecting a polypeptide in a biological sample comprising the step of contacting a biological sample suspected of containing the polypeptide with an antibody of claim 1 or 2 and detecting the formation of an antibody-antigen complex, wherein the formation of the antibody-antigen complex is indicative of the presence of the polycystin-related polypeptide.

12. A method as claimed in claim 11 in which the biological sample is selected from the group comprising: body fluid; solid tissue; tissue culture or cells derived therefrom; and sections of smears prepared from any of these sources.

13. A diagnostic kit for detecting a polypeptide present in a sample, comprising an antibody of any of Claims 1 or 2.

## Patentansprüche

1. Isolierter Antikörper, der gegen ein Epitop erzeugt wurde, wobei das Epitop die Aminosäuresequenz aufweist, die aus den Aminosäureresten 843 bis 1200, den Aminosäureresten 1205 bis 1625 oder den Aminosäureresten 1626 bis 2136 von SEQ ID Nr. 2 besteht.

2. Isolierter Antikörper gemäß Anspruch 1, wobei der Antikörper aus einem polyklonalen Antikörper, einem monoklonalen Antikörper oder einem mit einem nachweisbaren Marker markierten polyklonalen oder monoklonalen Antikörper ausgewählt ist.

3. Zusammensetzung, die einen Träger und einen Antikörper gemäß einem der Ansprüche 1 bis 2 umfasst.

4. Hybridomzelllinie, die einen monoklonalen Antikörper gemäß Anspruch 2 produziert.

5. Isolierter Antikörper gemäß einem der Ansprüche 1 bis 2, wobei das Epitop in einem Gewebe exprimiert wird, das aus der Gruppe ausgewählt ist, die aus Nieren-, Hirn-, Leber- und neuronalem Gewebe besteht.

6. Rekombinantes Polypeptid, das die Aminosäuresequenz aufweist, die aus den Aminosäureresten 843 bis 1200, den Aminosäureresten 1205 bis 1625 oder den Aminosäureresten 1626 bis 2136 von SEQ ID Nr. 2 besteht.

7. Zusammensetzung, die einen Träger und ein Polypeptid gemäß Anspruch 6 umfasst.

8. Isoliertes Polynucleotid, das das rekombinante Polypeptid gemäß Anspruch 6 codiert.

9. Gentransfervehikel, das das Polynucleotid gemäß Anspruch 8 umfasst.

10. Wirtszelle, die mit dem isolierten Polynucleotid gemäß Anspruch 8 transformiert ist.

11. Verfahren zum Nachweisen eines Polypeptids in einer biologischen Probe, umfassend den Schritt des In-Kontakt-Bringens einer biologischen Probe, von der man annimmt, dass sie das Polypeptid enthalten könnte, mit einem Antikörper gemäß Anspruch 1 oder 2 und des Nachweisens der Bildung eines Antikörper-Antigen-Komplexes, wobei die Bildung des Antikörper-Antigen-Komplexes die Anwesenheit des Polycystinzugehörigen Polypeptids anzeigt.

12. Verfahren gemäß Anspruch 11, wobei die biologische Probe aus der Gruppe ausgewählt ist, die Folgendes umfasst: Körperflüssigkeit; festes Gewebe; Gewebekultur oder daraus abgeleitete Zellen; und Schnitte oder Abstriche, die von einer dieser Quellen hergestellt wurden.

13. Diagnostischer Kit zum Nachweisen eines in einer Probe vorhandenen Polypeptids, der einen Antikörper gemäß einem der Ansprüche 1 oder 2 umfasst.

## Revendications

1. Anticorps isolé dirigé contre un épitope, l'épitope ayant la séquence d'acides aminés constituée des résidus d'acides aminés 843 à 1200, des résidus d'acides aminés 1205 à 1625, ou des résidus d'acides aminés 1626 à 2136, de la SEQ ID NO : 2.

2. Anticorps isolé selon la revendication 1, lequel anticorps est choisi parmi un anticorps polyclonal, un anticorps monoclonal, ou un anticorps polyclonal ou monoclonal marqué avec un marqueur détectable.

3. Composition comprenant un véhicule et un anticorps de l'une quelconque des revendications 1 et 2.

4. Lignée cellulaire d'hybridome qui produit un anticorps monoclonal tel que revendiqué dans la revendication 2.

5. Anticorps isolé selon l'une quelconque des revendications 1 et 2, dans lequel l'épitope est exprimé dans un tissu choisi dans le groupe constitué par les tissus des reins, du cerveau, du foie, et neuronaux.

6. Polypeptide recombiné ayant la séquence d'acides aminés constituée des résidus d'acides aminés 843 à 1200, des résidus d'acides aminés 1205 à 1625, ou des résidus d'acides aminés 1626 à 2136, de la SEQ ID NO : 2.

7. Composition comprenant un véhicule et un polypeptide de la revendication 6.

8. Polynucléotide isolé codant le polypeptide recombiné de la revendication 6.

9. Véhicule de délivrance de gène comprenant le polynucléotide de la revendication 8.

10. Cellule hôte transformée avec le polynucléotide isolé de la revendication 8.

11. Procédé de détection d'un polypeptide dans un échantillon biologique, comprenant l'étape consistant à mettre en contact un échantillon biologique suspecté de contenir le polypeptide avec un anticorps de la revendication 1 ou 2, et détecter la formation d'un complexe anticorps-antigène, dans lequel la formation du complexe anticorps-antigène est indicative de la présence du polypeptide apparenté à une polycystine.

12. Procédé selon la revendication 11, dans lequel l'échantillon biologique est choisi dans le groupe comprenant : un fluide corporel ; un tissu solide ; une culture tissulaire ou des cellules en dérivant ; et des sections de frottis préparés à partir de l'une quelconque de ces sources.

13. Trousse de diagnostic pour détecter un polypeptide présent dans un échantillon, comprenant un anticorps de l'une quelconque des revendications 1 ou 2.
